# FASCICULE DE BREVET EUROPEEN

(11) **EP 1 543 730 B1**
(45) Date de publication et mention de la délivrance du brevet: **15.08.2007**
(21) Numéro de dépôt: 04292989.3
(22) Date de dépôt: 14.12.2004
(51) Int. Cl.: A23K 1/16, A23K 1/18, C09J 189/00, A23K 1/00, C08L 89/00

(54) **Procédé de reticulation de protéines par un cétose de 3 à 5 atomes de carbone**
Vernetzungsverfahren von Proteinen mit Ketosen enthaltend 3 bis 5 Kohlenstoffatomen
Cross-linking process of proteins with a ketose containing 3 to 5 carbon atoms

(30) Priorité: 16.12.2003 FR 0314745
(43) Date de publication de la demande: 22.06.2005
(73) Titulaire: Roquette Frères, 62136 Lestrem (FR)
(72) Inventeur: Wils, Daniel, 59190 Morbecque (FR); Fouache, Catherine, 62113 Sailly Labourse (FR)
(74) Mandataire: Touati, Catherine

(56) Documents cités:
- EP-A- 0 284 548
- DYER D G ET AL: "FORMATION OF PENTOSIDINEDURING NONENZYMATIC BROWNING OF PROTEINS BYGLUCOSE IDENTIFICATION OF GLUCOSE AND OTHER CARBOHYDRATES AS POSSIBLE PRECURSORS OF PENTOSIDINE IN VIVO" JOURNAL OF BIOLOGICAL CHEMISTRY, AMERICAN SOCIETY OF BIOLOGICAL CHEMISTS, BALTIMORE, MD, US, vol. 266, no. 18, 25 juin 1991 (1991-06-25), pages 11654-11660, XP000673334 ISSN: 0021-9258
- TESSIER F J ET AL: "Triosidines : novel Maillard reaction products and cross-links from the reaction of triose sugars with lysine and arginine residues" BIOCHEMICAL JOURNAL, THE BIOCHEMICAL SOCIETY, LONDON, GB, vol. 369, 1 février 2003 (2003-02-01), pages 705-709, XP002287743 ISSN: 0264-6021
- SWAMY M S ET AL: "Glycation Mediated Lens Crystallin Aggregation and Cross-linking by Various Sugars and Sugar Phosphates In Vitro" EXPERIMENTAL EYE RESEARCH, ACADEMIC PRESS LTD., LONDON, GB, vol. 56, no. 2, 1 février 1993 (1993-02-01), pages 177-185, XP002287744 ISSN: 0014-4835

## Description

La présente invention est relative à un procédé de réticulation des protéines selon lequel l'agent de réticulation est un cétose de 3 à 5 atomes de carbone.

L'invention concerne également un procédé de réticulation, par au moins un cétose de 3 à 5 atomes de carbone, de protéines destinées à l'alimentation des animaux, à la fabrication de compositions filmogènes pour revêtements, enrobages ou adhésifs et à la préparation de matériaux protéiques biodégradables ou renouvelables.

Au sens de l'invention, on entend par « cétose de 3 à 5 atomes de carbone », un cétose choisi dans le groupe constitué par la dihydroxyacétone (ou la 1,3-dihydroxy-2-propanone), l'érythrulose, le xylulose et le ribulose.

L'invention concerne plus particulièrement l'utilisation de la dihydroxyacétone pour la réticulation de protéines.

On entend également par « agents réticulants » ou « agents de tannage » des protéines, des composés susceptibles de former des liaisons hydrogènes, coordinatives, covalentes ou ioniques avec lesdites protéines de manière à en réduire la digestibilité et la solubilité dans l'eau.

Trois familles principales d'agents réticulants de protéines sont généralement décrites.

La première famille est constituée par les tannins minéraux, tels les sels de cations di, tri ou polyvalents comme les sels de chrome, d'aluminium, de fer, de zinc, de cuivre, de cobalt, de titane, de zirconium, de silicium et de césium.

Les tannins minéraux sont surtout efficaces en milieu alcalin. Sous leur forme basique, ces sels de métaux polyvalents se lient de façon stable avec les fonctions carboxyliques libres de certains acides aminés des protéines.

Leur domaine d'application est en particulier celui du tannage de matériaux protéiques biodégradables ou renouvelables, notamment celui de la fabrication du cuir par tannage du collagène constituant le derme de la peau des animaux.

Cette opération a pour but de transformer la peau en cuir, c'est-à-dire de transformer une matière putrescible en une matière imputrescible, en ôtant aux microorganismes la possibilité de s'attaquer aux fibres du collagène.

Dans le cas du tannage au chrome qui représente environ 90 % des peaux tannées dans le monde, les composés de chrome se fixent sur les protéines en formant des complexes :
- soit avec les groupes carboxyliques libres des chaînes aminées desdites protéines,
- soit par l'intermédiaire de groupes sulfates, formiates ou oxalates, avec les fonctions amines libres des chaînes aminées desdites protéines.

Une réticulation, i.e. une liaison de deux ou plusieurs chaînes peptidiques, par l'intermédiaire des composés du chrome, a alors lieu.

Ces tannins minéraux sont particulièrement efficaces, mais génèrent de grandes quantités de déchets préjudiciables pour l'environnement.

Les tannins minéraux sont parfois également utilisés dans le domaine du tannage des protéines destinées à l'alimentation animale, sous la forme de sels de zinc ou d'autres cations. Une consommation excessive de telles protéines tannées est à éviter car elle peut conduire à des effets toxiques pour l'animal.

La deuxième famille de tannins est constituée par les tannins synthétiques tels les résines épichlorhydrines, les résines polyamide-amine-épichlorhydrines, les résines polyéthylène-imines et les résines isocyanates comme le méthylènediphényl diisocyanate.

Leur domaine d'application est en particulier celui des colles ou des adhésifs, notamment des colles à bois ou des colles pour carton ondulé.

La troisième famille de tannins est constituée par les tannins aldéhydiques, plus particulièrement le formaldéhyde, le glyoxal (éthanedial) ou le glutaraldéhyde, et l'ensemble de leurs dérivés commerciaux vendus sous forme de condensats divers.

Les tannins aldéhydiques se lient aux protéines par le biais de liaisons covalentes en générant principalement des produits de condensation. Une réaction de double condensation peut également conduire à la formation de produits réticulés.

Ces produits de condensation sont généralement issus de la réaction de formation d'imines par condensation de la fonction carbonyle de l'agent réticulant avec la fonction amine libre de certains acides aminés de ladite protéine (notamment la fonction ε-aminée de la lysine).

Les tannins aldéhydiques sont en particulier utilisés dans trois principaux domaines d'application.

Le premier domaine d'application est celui de la préparation de matériaux protéiques, par exemple des cuirs, des pièces moulées, des fibres textiles, des capsules pharmaceutiques ou alimentaires, des films photographiques, des pièces automobiles et des boutons, à partir de caséines de lait, de zéines, de collagènes, de protéines de soja, de sang d'abattoir, de gélatines, réticulées le plus souvent au formaldéhyde.

Pour les cuirs, le tannage par les agents aldéhydiques consolide la structure du collagène en créant des liaisons entre des groupements du tannin aldéhydique et au moins deux groupements appartenant à la chaîne peptidique. Des cuirs particuliers peuvent être ainsi obtenus.

Le deuxième domaine d'application est celui du tannage des protéines destinées à la fabrication de compositions filmogènes pour revêtements, enrobages ou adhésifs.

On utilise par exemple couramment pour le collage de papiers, de cartons, de particules, de bois ou d'autres matériaux, des colles préparées à partir de gélatine, de protéines de sang, de protéines de poisson, de caséines de lait et de protéines végétales, dans lesquelles est ajouté généralement du formaldéhyde ou du glyoxal, afin d'améliorer la résistance à l'eau et à l'humidité du joint de colle ainsi que ses caractéristiques adhésives.

Le troisième domaine d'application est celui du tannage des protéines destinées à l'alimentation notamment des ruminants, pour la fabrication de protéines dites « résistantes ».

Les protéines résistantes sont alors des protéines modifiées de telle manière à ce qu'elles soient moins dégradées par certaines enzymes microbiennes dans le rumen.

Cependant, après passage dans le rumen, ces protéines retrouvent une digestibilité satisfaisante quand elles arrivent dans la « caillette » et dans l'intestin grêle. Le pH acide et les enzymes de la digestion libèrent en effet les protéines et les rendent ainsi à nouveau disponibles.

Cette opération de tannage autorise donc une meilleure assimilation de la ration alimentaire par les ruminants, conduisant à de meilleures performances zootechniques (amélioration des productions de viande et de lait).

Cependant, l'inconvénient majeur des tannins aldéhydiques utilisés dans ces trois domaines d'application, est leur forte toxicité.

L'un des aldéhydes les plus couramment utilisés, parce que très réactif, est le formaldéhyde (encore appelé formol sous son nom d'usage technique). Or, cet agent chimique est parmi les plus toxiques et les plus dangereux des tannins aldéhydiques à manipuler, car il est à l'état gazeux à température ambiante.

Dans le domaine de l'alimentation des ruminants, pour remédier à cet usage de substances actives, mais trop toxiques, il a été proposé par exemple dans le brevet EP 284.548 de mettre en oeuvre une réaction de tannage au moyen d'une catégorie particulière d'aldéhydes, e.g. les aldoses.

Il s'agit ici du xylose, du ribose, du mannose, du lactose et du glucose. Ces molécules sont non toxiques, contrairement aux tannins aldéhydiques classiquement utilisés.

Cependant, l'enseignement de ce brevet révèle qu'il est nécessaire de contrôler finement la réaction de tannage de manière à obtenir ce que les auteurs de ce brevet appellent un produit de condensation de Maillard « précoce » plutôt qu'un produit de Maillard « intermédiaire ».

Il est en outre indispensable de sélectionner un aliment qualifié « d'orthodoxe », i.e. contenant un groupe de protéines à fonction amine libre présent dans l'aliment dans un rapport 1,5 à 1 par rapport aux protéines totales.

Il faut enfin hydrolyser le produit de condensation à un pH inférieur à 4, et en limiter le pourcentage de sucres réducteurs.

Le procédé décrit dans le brevet EP 284.548 est donc bien trop lourd à mettre en oeuvre.

A côté des aldoses proposés dans le brevet EP 284.548, un seul cétose est évoqué dans ladite demande de brevet. Il s'agit du fructose.

Cependant, ce cétose à 6 atomes de carbone n'est considéré qu'en raison de son pouvoir réducteur intermédiaire entre ceux du glucose et du mannose. Ce brevet EP 284.548 ne fait que citer ce cétose sans le recommander véritablement. Aucun autre cétose n'est mis en oeuvre dans le procédé décrit.

Par ailleurs, à la meilleure connaissance de la société Demanderesse, les potentialités et les réactivités chimiques d'autres sucres porteurs d'une fonction cétone n'ont jamais fait l'objet d'une exploration pour les domaines d'activité concernés par la présente invention.

Le cétose le plus simple de la série des sucres est la dihydroxyacétone (ou DHA), également appelée 1,3-dihydroxy-2-propanone, qui est un composé de formule CH₂OH-CO-CH₂OH.

A côté de son utilisation dans le secteur de la cosmétique, mais en applications externes pour ses propriétés de coloration de l'épiderme de la peau (effet « auto bronzant ») - utilisée seule ou associée à l'érythrulose - la DHA est surtout connue pour ses propriétés anti-oxydante, inhibitrice d'odeur et biocide (effet antibactérien et effets antibactérien et antifongique).

La DHA est surtout préconisée en mélange avec l'acide pyruvique, notamment pour assurer le contrôle métabolique du contenu en lipides du foie et des adipocytes. Il est alors possible de contrôler par ce biais la balance lipides / protéines chez les mammifères.

Cette utilisation est par exemple décrite dans le brevet US 4.351.835 par l'administration orale de pyruvate et de DHA afin d'éviter la prise de poids chez les mammifères, ou dans le brevet US 4.415.575, où leur administration favorise la concentration en protéines chez les animaux.

Pour illustrer une autre propriété connue de la DHA, i.e. son effet inhibiteur d'odeurs indésirables, il est décrit dans la demande de brevet JP 50.7803 l'utilisation de la DHA dans la prévention de la résurgence des odeurs d'huiles et de graisses.

L'effet biocide de la DHA est quant à lui par exemple décrit dans le brevet CA 1.054.434. Elle est utilisée comme additif alimentaire, notamment pour la préservation d'aliments (poissons, viandes, fruits, légumes...) par mise en contact à température ambiante de l'aliment avec de la DHA.

Quant aux cétoses à 4 ou 5 atomes de carbone (i. e. érythrulose, ribulose ou xylulose), contrairement à la DHA, il ne semble pas selon la littérature qu'ils aient fait l'objet de travaux d'étude de leurs propriétés intrinsèques.

Ces dernières molécules sont à la connaissance de la société Demanderesse seulement utilisées aujourd'hui comme intermédiaires de synthèse de produits à valeur ajoutée, tels leurs produits d'hydrogénation correspondants (érythritol, ribitol, arabitol et xylitol).

De tout ce qui précède, il résulte qu'il existe un besoin non satisfait de disposer d'un substitut d'agents réticulants, notamment d'un substitut des tannins minéraux, de tannins synthétiques et de tannins aldéhydiques, simple à produire et à mettre en oeuvre, d'une efficacité équivalente, voire améliorée, par rapport aux agents de tannage classiques.

La société Demanderesse a donc eu le mérite, au terme de recherches longues et fastidieuses, de proposer d'utiliser au moins un cétose de 3 à 5 atomes de carbone comme substitut d'agents réticulants de protéines.

Rien ne suggérait dans l'état de la technique, que l'utilisation d'un cétose de 3 à 5 atomes de carbone puisse convenir au tannage des protéines, et remplacer tout ou partie des agents réticulants habituels des protéines, notamment dans des domaines aussi variés que le tannage de protéines destinées à l'alimentation des animaux, la fabrication de compositions filmogènes pour revêtements, enrobages ou adhésifs et la préparation de matériaux protéiques biodégradables ou renouvelables.

Lesdites protéines sont alors choisies dans le groupe comprenant les protéines de tissus animaux, du lait, du sang, comme notamment la caséine, la gélatine, le collagène ; les protéines de céréales, comme notamment les protéines de maïs, de blé, de riz ; les protéines de protéagineux, comme notamment les protéines de pois, de luzerne, de lupin, d'orge, de millet, de sorgho ; les protéines d'oléagineux, comme notamment les protéines du soja, tels les tourteaux de soja, du colza, de lins, tels les tourteaux de colza, du tournesol, des arachides, du coton ; et les protéines de tubercules comme notamment de la pomme de terre, du manioc.

Dans la présente demande, on entend par « protéines de tissus animaux », les protéines issues de tissus de tous les animaux à l'exception de l'homme.

Selon un mode de réalisation avantageux, les protéines sont choisies dans le groupe comprenant les protéines de céréales, comme notamment les protéines de maïs, de blé, de riz ; les protéines de protéagineux, comme notamment les protéines de pois, de luzerne, de lupin, d'orge, de millet, de sorgho ; les protéines d'oléagineux, comme notamment les protéines du soja, tels les tourteaux de soja, du colza, de lins, tels les tourteaux de colza, du tournesol, des arachides, du coton ; et les protéines de tubercules comme notamment de la pomme de terre, du manioc.

De préférence, les agents réticulants substitués totalement ou partiellement par au moins un cétose de 3 à 5 atomes de carbone sont choisis dans le groupe des tannins minéraux, des tannins synthétiques et des tannins aldéhydiques.

Le cétose de 3 à 5 atomes de carbone selon l'invention est choisi dans le groupe constitué par la dihydroxyacétone, l'érythrulose, le ribulose et le xylulose, et est préférentiellement la dihydroxyacétone.

La société Demanderesse a trouvé que la réactivité du cétose de 3 à 5 atomes de carbone utilisé comme substitut d'agents réticulants est fonction de son poids moléculaire (i.e. de son nombre de carbone). Les cétoses choisis peuvent ainsi être classés de l'espèce la plus réactive (DHA à 3 atomes de carbone) à l'espèce la moins réactive (xylulose et ribulose à 5 atomes de carbone).

Le choix de l'un ou l'autre cétose sera donc fonction du degré de réticulation souhaité ou du domaine d'application visé.

Selon un premier mode d'utilisation d'un cétose de 3 à 5 atomes de carbone, la société Demanderesse a démontré qu'un tel cétose pouvait convenir efficacement et être recommandé pour réticuler des protéines destinées à l'alimentation des animaux, notamment des ruminants ou des animaux de compagnie, ou en aquaculture.

Celles-ci peuvent être choisies en particulier dans le groupe constitué par les protéines animales, de céréales, de protéagineux, d'oléagineux et de tubercules, comme notamment les tourteaux de soja, les tourteaux de colza, les tourteaux de lins, les protéines du lait, les protéines du pois, de la luzerne, les protéines du tournesol, du lupin, des arachides, du coton, de l'orge, du millet, du sorgho, les protéines du maïs, du blé (notamment le gluten) et du riz, les protéines de la pomme de terre et du manioc.

Il peut être utilisé de manière indifférenciée chacun des quatre cétoses de 3 à 5 atomes de carbone comme agents de substitution des tannins aldéhydiques.

La société Demanderesse recommande cependant d'utiliser préférentiellement la DHA comme substitut du formol pour le tannage des protéines destinées à l'alimentation des animaux, notamment des ruminants ou des animaux de compagnie, ou en aquaculture.

Le choix de la DHA comme substitut d'un agent de tannage aldéhydique est en soi remarquable, car à la meilleure connaissance de la société Demanderesse, la DHA n'a jamais été décrite pour le tannage des protéines destinées à l'alimentation du bétail.

En effet, la réticulation des protéines vise ici l'obtention de protéines résistantes, de protéines moins solubles et moins biodisponibles dans le rumen, alors que la DHA n'est jusqu'à présent utilisée que pour ses propriétés auto-bronzantes.

Il est important de noter que les propriétés réticulantes de la DHA, classiquement mise en oeuvre en Cosmétique, voire parfois évoquée dans les processus pathologiques tels les complications du diabète ou dans le vieillissement des protéines, ne sont pas concernées par la présente invention.

Par rapport aux protéines natives à partir desquelles elles sont préparées, la société Demanderesse a démontré que les condensats de protéines résistantes obtenues par un traitement par la DHA présentent un contenu en acides aminés modifié, plus particulièrement en certains de leurs acides aminés à fonction amine libre, telles la lysine, l'arginine, la glutamine et l'asparagine.

La société Demanderesse a trouvé que les acides aminés se retrouvent dans la protéine sous la forme de produits modifiés de condensation simple, voire double avec la DHA.

La DHA se condense préférentiellement par exemple avec la lysine et l'arginine en formant des imines. Une double condensation peut conduire à des produits réticulés.

Par ailleurs, la société Demanderesse a démontré que la présence du groupement hydroxylé en alpha de la fonction carbonyle de la DHA conduit à ce que l'imine obtenue par condensation sur un acide aminé terminal puisse se cycliser avec la fonction carboxylique de cet acide pour former une δ-lactone.

Des études comparatives menées par la société Demanderesse sur la modification des protéines par le formol ont montré également que parmi tous les acides aminés constitutifs desdites protéines, c'est plutôt la tyrosine qui est modifiée par les réactions de condensation avec le formol, alors que le contenu en lysine et en arginine n'est pas modifié.

Sans être lié par une quelconque théorie, il apparaît que c'est cette condensation des cétoses sur la fonction amine libre des acides aminés notamment de type lysine et arginine (pour les tourteaux de colza et de soja), de type glutamine (pour le gluten de blé) qui permet d'expliquer la résistance que présentent les condensats de protéines conformes à l'invention aux attaques enzymatiques et microbiennes subies par lesdites protéines par exemple dans le rumen.

En effet, la condensation sur ces acides aminés peut empêcher l'accessibilité des enzymes de dégradation aux protéines, par encombrement stérique ou par modification locale de la configuration desdites protéines.

Les protéines choisies dans le groupe constitué par les tourteaux de soja, les tourteaux de colza, les tourteaux de lin, les protéines du lait, les protéines du pois et du blé (gluten) se prêtent particulièrement bien au tannage par la DHA.

Selon un deuxième mode d'utilisation des cétoses de 3 à 5 atomes de carbone, la société Demanderesse a montré qu'il est possible de substituer avantageusement les réticulants habituels des protéines utilisées pour la préparation de compositions filmogènes pour revêtements, enrobages ou adhésifs.

Ces protéines peuvent alors être choisies dans le groupe constitué par les protéines de céréales, de tubercules, d'oléagineux, de protéagineux, de lait, de sang et de tissus animaux, préférentiellement la caséine, la gélatine, la zéine, et les isolats de protéagineux.

La société Demanderesse recommande également d'utiliser préférentiellement la DHA. En effet, la DHA présente de remarquables effets insolubilisants et réticulants de protéines, même à température ambiante, que ce soit à pH neutre ou alcalin, comme il sera exemplifié ci-après. Elle permet de ce fait d'améliorer les propriétés mécaniques des films protéiques, tout en ajustant leur résistance à l'eau.

La société Demanderesse tient également à insister sur l'intérêt du caractère progressif dans le temps de l'effet réticulant de la DHA, caractère qu'elle a démontré.

La DHA convient donc tout particulièrement aux domaines d'application visés, contrairement au formol dont le caractère réticulant instantané induit une prise trop rapide de viscosité.

La réactivité lente de la DHA permet de ce fait d'envisager l'emploi de la DHA dans la formulation de compositions destinées à préparer des films d'emballage, de protection, d'enrobage et de surfaçage, plus ou moins solubles dans l'eau.

Il peut s'agir de compositions destinées au domaine de la détergence (enrobage de poudres de parfums, d'enzymes...), du traitement de surfaces papetières (papiers, cartons...) de la métallurgie (tôles...), de la construction (enrobage de ciments ou de pigments, films antigraffitis...), de l'agriculture (enrobage de semences, formes retard d'engrais ou de produits phytosanitaires...), vétérinaire (enrobage de vitamines, d'oligo-éléments...), pharmaceutique (enrobage ou encapsulation d'actifs) et alimentaire (films barrière, films comestibles, enrobage de produits alimentaires, notamment produits de charcuterie et produits de confiserie).

Il peut s'agir également de compositions destinées à préparer des colles nécessitant une très bonne tenue à l'eau et/ou une excellente adhésivité (liant d'agglomération de particules minérales ou organiques, liant de pigments, colle papiers peints, colle d'étiquetage, colle pour bois, liège, matériaux fibreux).

Les compositions filmogènes pour revêtements, enrobages ou adhésifs obtenues selon l'invention peuvent comprendre entre autres, différents additifs tels que des plastifiants, des agents conservateurs, antioxygène, hydrophobes, anticorrosion, des colorants, des parfums ainsi que des principes actifs.

Selon un troisième mode d'utilisation des cétoses de 3 à 5 atomes de carbone, la société Demanderesse a démontré la possibilité de réticuler des protéines destinées à la préparation de matériaux protéiques biodégradables ou renouvelables, tels que des films plastiques, des cuirs, des fibres textiles, des gélules, des capsules, ou des compositions protéiques alimentaires.

Les protéines peuvent alors être avantageusement choisies dans le groupe constitué par les protéines de céréales, de tubercules, d'oléagineux, de protéagineux, de lait, de sang et de tissus animaux, préférentiellement le collagène, la caséine, la gélatine, la zéine, et les isolats de protéagineux.

Il peut s'agir de compositions destinées à l'industrie de l'emballage (films protéiques), l'industrie photographique, l'industrie pharmaceutique (capsules et gélules), l'industrie du caoutchouc (additifs pour pneus), l'industrie textile (fibres protéiques) et à la plasturgie (objets protéiques moulés ou extrudés).

Par ailleurs, la société Demanderesse recommande tout particulièrement d'utiliser la DHA comme substitut de tannins minéraux, tel le chrome, ou de tannins aldéhydiques, tel le formol, pour le tannage du cuir. Les autres cétoses de 4 et 5 atomes de carbone sont également utilisables bien que moins réactifs.

Pour réticuler les protéines à l'aide d'un cétose de 3 à 5 atomes de carbone selon l'invention, on réalise la succession des étapes suivantes qui consiste à :
1) mettre en contact les protéines et au moins un cétose de 3 à 5 atomes de carbone, à une concentration en poids sec du cétose de 0,5 à 20 %, de préférence de 0,5 à 10 % et plus préférentiellement de 0,5 à 5 % par rapport au poids sec des protéines brutes à traiter,
2) maintenir le contact entre les protéines et le cétose pendant une durée supérieure à quelques minutes, de préférence comprise entre 10 minutes et 60 minutes, de préférence pendant 30 minutes, à une température comprise entre 20 et 105°C, de préférence comprise entre 35 et 105°C,
3) et récupérer les protéines réticulées ainsi obtenues.

Ce procédé de base est valable quelle que soit la nature du cétose de 3 à 5 atomes de carbone choisi, la protéine à traiter et le domaine d'application visé.

La société Demanderesse recommande cependant de faire varier le couple temps / température en fonction du cétose choisi en regard du degré de réticulation visé, comme il sera décrit et exemplifié ci-après.

Pour la réticulation à la DHA de protéines destinées à l'alimentation des animaux, la société Demanderesse recommande, dans la première étape du procédé, de mettre en contact les protéines et par exemple la DHA à une concentration en poids sec de DHA de 0,5 à 10 % et plus préférentiellement de 0,5 à 5 % par rapport au poids sec de la protéine brute à traiter.

Les protéines ainsi mises en contact sont particulièrement choisies dans le groupe constitué des tourteaux de soja, les tourteaux de colza, les tourteaux de lin, les protéines du pois, le gluten soluble, le gluten de blé natif et le lactosérum, comme il sera exemplifié ci-après.

La température de mise en contact dans un mélangeur ou un pétrin à retenir est alors plus particulièrement de 45 à 100°C pendant 30 minutes.

Les protéines tannées à la DHA ainsi obtenues pour ce domaine sont analysées selon l'invention en termes de :
- contenu en azote soluble / azote total, exprimé en %/ sec qui traduit la part de protéines qui n'a pas réagi avec la DHA, par la méthode décrite dans l'article de R. VERITE et C. DEMARQUILLY intitulé *Qualité des matières azotées des aliments pour ruminants,* dans la revue *La vache Laitière,* publication INRA 1978, p 154,
- composition en acides aminés totaux, déterminée par une méthode mise au point par la société Demanderesse.

Cette méthode de détermination de la composition en acides aminés totaux consiste en une hydrolyse acide de 24 heures des protéines et analyse des acides aminés libérés par chromatographie liquide haute pression sur résine échangeuse d'ions.

Pour l'hydrolyse des protéines, on calcule tout d'abord la pesée d'échantillon qui doit contenir 2,8 mg d'azote dans 14 ml d'HCl 6N.

On ajoute ensuite à ladite pesée 14 ml d'HCl 6 N et place sous vide pendant 5 minutes.

Après fermeture du tube, on le place à 115 °C pendant 24 h.

On ajoute ensuite 1 ml de Norleucine 10 mM (commercialisée par la société SIGMA) qui servira de témoin d'élution, et filtre la solution refroidie sur fritté.

On récupère le filtrat dans un ballon de 250 ml et évapore à sec à + 4°C. On lave le résidu avec 100 ml d'eau et évapore à sec, puis reproduit le lavage deux fois avec 50 ml d'eau et sèche à nouveau.

On récupère enfin le résidu sec dans 50 ml du tampon lithium Li 280 (commercialisé par la société LC Tech).

Les analyses chromatographiques en CLHP sont effectuées sur colonne échangeuse de cations (colonne référencée 0353150 de la société PICKERING).

L'étalonnage de la colonne est effectué avec 100 µl d'une solution standard d'acides aminés (commercialisée par la société SIGMA sous la référence AAS18) et 20 µl de la solution de Norleucine.

100 µl de la solution des protéines hydrolysées sont ensuite injectés sur ladite colonne, et les acides aminés sont élués à l'aide d'une solution de ninhydrine à 0,3 ml/min.

Les acides aminés détectés par UV à 570 nm sont identifiés et quantifiés sur le chromatogramme obtenu.

Comme il sera exemplifié ci-après, la détermination des compositions en acides aminés totaux est effectuée sur des tourteaux de soja traités ou non à la DHA, en les comparant à la composition en acides aminés totaux des mêmes tourteaux de soja traités au formol.

Quant à l'utilisation possible des autres cétoses de 4 à 5 atomes de carbone comme substituts d'agents tannants des protéines destinées à cette même application, elle est illustrée par la détermination de la réactivité du xylulose et du ribulose sur la lysine, à une température de 80°C, comme il sera également exemplifié ci-après.

Pour la réticulation à la DHA de protéines destinées à la préparation de films, notamment pour la réticulation de caséine, de gélatine ou isolats de protéagineux, la société Demanderesse recommande, dans la première étape du procédé, de mettre en contact la protéine et la DHA à une concentration en poids sec de DHA de 1 à 10 % et plus préférentiellement de 1 à 5 % par rapport au poids sec de la protéine brute à traiter.

La mise en contact s'effectue à température ambiante pendant quelques minutes, et les protéines tannées ainsi obtenues sont analysées en termes de viscosité de colles, d'effet insolubilisant, de propriétés des films obtenus (en particulier de résistance mécanique), comme il sera exemplifié ci-après.

Pour la préparation de cuirs tannés avec de la DHA, la succession des étapes 1 à 3 décrites plus haut convient également.

D'autres caractéristiques et avantages de l'invention apparaîtront à la lecture des exemples non limitatifs décrits ci-dessous.

### Exemple 1

On élabore 6 condensats de protéines tannées à la dihydroxyacétone à partir de tourteaux de soja (commercialisés par la société UNEAL), de tourteaux de colza (produits par les MOULINS DELSALLE), de protéines de pois (commercialisées par la société COSUCRA sous le nom de marque PISANE® HD), de gluten soluble (commercialisé par la société Demanderesse sous le nom de marque VITEN^{®} CWS), de gluten de blé natif (commercialisé par la société Demanderesse sous le nom de marque VITEN^{®}) et de protéines du lactosérum (commercialisées par la société ARMOR PROTEINES sous le nom de marque PROTARMOR^{®} 865).

La DHA peut-être utilisée pure (commercialisée par la société SIGMA) ou utilisée directement sous la forme d'un surnageant de fermentation de microorganismes en produisant à partir de glycérol coproduit du diester (DHA dite « technique »).

Si l'on choisit de la DHA pure, on procède dans un premier temps à la dilution de ladite DHA dans un volume d'eau calculé de manière à ce que le rapport DHA / Protéines en final correspondent à la valeur souhaitée.

Il s'agit généralement d'une solution de 10 à 25 % de matière sèche.

On attend 30 minutes afin de stabiliser la solution de DHA sous forme de monomères. On rectifie le pH à 6 avec de la soude 0,1 N.

On préchauffe les protéines à 100°C dans un pétrin pendant 30 minutes. La DHA est ajoutée, le mélange est homogénéisé et on réalise la mise en contact proprement dite des protéines avec la DHA à la température de 95 à 100°C pendant encore 30 minutes.

Les résultats du tableau I suivant permettent d'estimer les propriétés insolubilisantes de la DHA, par la détermination des rapports azote soluble / azote total des 6 condensats de protéines après tannage à la DHA aux doses de 1, 2,5 et 5 % en poids sec/ sec de protéines traitées.

**Tableau I**

| | Soja | Colza | Pois | VITEN^{®} CWS | VITEN^{®} | Lactosérum |
|---|---|---|---|---|---|---|
| Départ | 18 | 21 | 22 | 67 | 8,4 | 98 |
| 1% DHA | 5 | 14 | 8 | 52 | 3 | 24 |
| 2,5 % DHA | 3 | 5 | 3 | 53 | 1 | 16 |
| 5 % DHA | 3 | 6 | 5 | Nd | 1 | 10 |
| 1 % formaldéhyde | 5 | Nd | Nd | Nd | nd | nd |

Le rapport azote soluble / azote total diminue d'autant plus que la dose en DHA est élevée, mais un effet significatif est déjà obtenu à la dose de 1 %.

L'essai comparatif de tannage des tourteaux de soja avec le formol montre que l'effet d'insolubilisation des protéines réalisé par traitement à la DHA à la dose de 1% est équivalent à celui obtenu avec le formol à la dose de 1 %.

La DHA est donc un substitut du formol tout à fait efficace pour le tannage des protéines destinées à l'alimentation des ruminants, de quelque nature qu'elles puissent être.

Des essais en parallèle ont été menés sur tourteaux de colza et tourteaux de soja pour étudier l'effet de la température et du temps de contact.

Il est ainsi montré que la réaction s'effectue facilement, même à température ambiante, avec un résultat significatif dans les 10 premières minutes de contact.

Il a été également noté que le soja est plus réactif que le colza.

Le tableau II suivant présente le bilan en acides aminés déterminé sur protéines natives (colza et soja pris comme exemple), puis sur protéines traitées avec 1 % en DHA.

**Tableau II**

| | Soja | | Colza | | Soja | |
|---|---|---|---|---|---|---|
| Acides aminés | Natif | + DHA | Natif | + DHA | Natif | + formol |
| Asp | 11,8 | 11,9 | 8 | 8,2 | 11,8 | 12 |
| Thr | 4,2 | 4,1 | 5,1 | 5,2 | 3,95 | 3,8 |
| Ser | 5,3 | 5,4 | 4,8 | 5,2 | 5,3 | 4,9 |
| Glu | 19,9 | 20,2 | 20,1 | 20,5 | 19,9 | 20,2 |
| Gly | 4,4 | 4,6 | 5,7 | 5,8 | 4,4 | 4,45 |
| Ala | 4,4 | 4,4 | 4,8 | 5,1 | 4,4 | 4,45 |
| Val | 5,1 | 5,1 | 5,7 | 6,3 | 5,1 | 5,3 |
| Ile | 4,8 | 4,9 | 4,5 | 4,5 | 4,85 | 5,1 |
| Leu | 8,1 | 8,3 | 8 | 8,3 | 8,1 | 8,2 |
| **Tyr** | 3,9 | 4,1 | 3,4 | 3,5 | 4 | **3,5** |
| Phe | 5,3 | 5,4 | 4,5 | 4,5 | 5,3 | 5,3 |
| **Lys** | 6,5 | **5,8** | 6,4 | **5,6** | 6,5 | 6,4 |
| His | 2,7 | 2,7 | 3,1 | 3,1 | 2,8 | 2,7 |
| **Arg** | 8,1 | **7,3** | 8,3 | **6,9** | 8,1 | 8,2 |
| Pro | 5,5 | 5,8 | 7,6 | 7,3 | 5,5 | 5,5 |
| AA totaux | 100 | 100 | 100 | 100 | 100 | 100 |

Le tannage des protéines des tourteaux de soja et de colza avec 1 % de DHA conduit à une diminution de la teneur en lysine de l'ordre de 10,8 % (cas du soja) et de l'ordre de 12,7 % (cas du colza), et à une diminution de la teneur en arginine de l'ordre de 9,9 % (cas du soja) et de l'ordre de 16,9 % (cas du colza), indiquant par la-même que les réactions de condensation de ces protéines avec la DHA ciblent plus particulièrement les fonctions amines libres de ces deux acides aminés constitutifs des chaînes peptidiques.

En comparaison, les réactions de condensation du formol avec les protéines du soja se font principalement avec les résidus tyrosine des chaînes peptidiques. Il apparaît ainsi que la DHA, tout comme le formol se combine aisément avec les protéines destinées à l'alimentation des ruminants, avec une réactivité équivalente à celle du formol, la toxicité bien évidemment en moins.

### Exemple 2

On réalise la digestion des tourteaux de soja de l'exemple 1 traités ou non à la DHA à l'aide d'une combinaison d'une α-amylase (Solvay Amylase - LF60 commercialisée par la société SOLVAY ENZYMES), d'une cellulase (SPEZYME CP commercialisée par la société GENENCOR) et d'une pepsine (PEPSINE P-7000 commercialisée par la société SIGMA), afin de déterminer le degré de résistance des protéines ainsi obtenues.

Ce test *in vitro* permet d'évaluer l'efficacité de la réaction de tannage et la disponibilité en protéines après le by-pass du rumen. Cette digestion à la pepsine permet en outre de simuler le pH et la dégradation enzymatique de l'estomac.

Pour cette évaluation, deux séries de digestion sont alors réalisées en parallèle :
- une première série avec deux enzymes (protocole AC), i.e. α-amylase et cellulase, afin d'estimer l'efficacité de la réaction de tannage (protéines résistantes aux enzymes du rumen),
- une deuxième série avec les trois enzymes (protocole ACP), i.e. α-amylase, cellulase et pepsine (la digestion à la pepsine traduisant la digestibilité des protéines tannées après le passage dans le rumen, ici dans l'estomac).

La digestion enzymatique des protéines tannées ou non s'effectue en suivant le protocole suivant :
1) on pèse 2,5 g de produit sec,
2) on ajoute 25 ml d'HCl 0.075 N à pH 4,
3) on agite pendant 1 h à température ambiante (ajuster le pH à 4 si nécessaire),
4) on ajoute 200 µl d' α-amylase (à 60.000 MWU/ml.min) et 200 µl (à 90 GCU/ml) de cellulase,
5) on laisse sous agitation pendant 3 heures au bain-marie à 40°C,
6) on ajoute 25 ml d'HCl 0.075 N à pH 1,2,
7) on agite et vérifie le pH (ajuster le pH à 1,5 si nécessaire),
8) on ajoute à cette étape la pepsine (dans le cas du protocole ACP) à raison de 500 µl de pepsine à la concentration de 5 g/l, préparée extemporanément,
9) on place alors au bain-marie à 40°C et agite continuellement pendant 20 h,
10) on recueille le digestat, laisse décanter, prélève deux fois 15 ml du surnageant et centrifuge à 4000 rpm pendant 20 min,
11) on récupère le surnageant et peut le congeler pour stockage.

La détermination du degré d'hydrolyse des protéines est réalisée par une méthode de dosage des groupements aminés primaires des protéines traitées avec l'acide trinitrobenzène sulfonique (TNBS).

Cette méthode consiste à :
- ajouter 250 µl de SDS à 5 % (Sodium Dodecyl Sulfate) à 250 µl d'échantillon à analyser,
- laisser 30 min à 50°C,
- ajouter 2 ml de tampon phosphate à pH 8,2,
- ajouter 2 ml de TNBS à 1 %,
- placer au bain-marie à 50°C pendant 1 h,
- ajouter 4 ml d'HCl 0,1 M,
- laisser 30 min à température ambiante,
- prélever 250 µl et lire sur spectrophotomètre à 340 nm.

Une gamme étalon est réalisée avec une solution de leucine à 10 mM.

Les résultats de digestion enzymatique sur les échantillons peuvent être exprimés en concentration en équivalent leucine (EL) exprimée en mMol/l ou en % (baisse de la réponse TNBS par rapport à la protéine native).

Le tableau III suivant présente les valeurs de digestibilité obtenues pour les tourteaux de soja traités avec des doses variables en DHA (1 et 2,5 %) suivant la mise en oeuvre décrite dans l'exemple 1.

**Tableau III**

| Concentration en DHA | Sans traitement enzymatique | | Protocole AC | | Protocole ACP | |
|---|---|---|---|---|---|---|
| | EL | % | EL | % | EL | % |
| 0 | 2,40 | 100 | 3,17 | 100 | 17,03 | 100 |
| 1 % | 1,49 | **62,2** | 1,97 | **62,0** | 14,61 | **85,8** |
| 2,5 % | 0,53 | 22,1 | 0,68 | 21,4 | 9,79 | 57,5 |

On constate que plus la dose en DHA est importante, moins les tourteaux sont sensibles à la digestion par l' α-amylase et la cellulase (traduisant ainsi une bonne protection dans le rumen), tout en permettant une bonne sensibilité à la pepsine qui traduit une biodisponibilité satisfaisante (après le by-pass du rumen).

A la dose de 1 % en DHA, les protéines tannées ne sont pratiquement pas digérées (la valeur du % de réponse passe de 62,2 à 62 %). Cependant, l'ajout de la pepsine rend disponible plus de 85 % desdites protéines.

Toutefois, il est à noter que la dose de 2,5 % de DHA présente un effet protecteur important, puisque après traitement à la pepsine seulement 57,5 % des protéines sont disponibles.

La dose de 1 % de DHA est donc à retenir pour le tannage des protéines destinées à l'alimentation des ruminants.

Des résultats équivalents sont obtenus avec les tourteaux de colza, comme l'indique le tableau IV suivant.

**Tableau IV**

| Concentration en DHA | Sans traitement enzymatique | | Protocole AC | | Protocole ACP | |
|---|---|---|---|---|---|---|
| | EL | % | EL | % | EL | % |
| 0 | 1,60 | 100 | 2,13 | 100 | 8,40 | 100 |
| 1 % | 1,32 | 82,8 | 1,87 | 87,7 | 7,36 | 87,6 |
| 2,5 % | 0,81 | 50,9 | 1,16 | 54,4 | 5,16 | 61,4 |

On confirme ainsi que le procédé de tannage à la DHA est plus efficace pour les tourteaux de soja que pour les tourteaux de colza (cf une des conclusions de l'exemple 1).

### Exemple 3

On réalise ici la digestion des tourteaux de soja de l'exemple 1 traités ou non à la DHA à l'aide de la combinaison d'enzymes de l'exemple 2 à laquelle on ajoute de la trypsine (Trypsine T 8003 issue de pancréas de bovin commercialisée par la société SIGMA).

Ce test *in vitro* à la trypsine permet de simuler les attaques enzymatiques de l'intestin grêle sur les tourteaux traités ou non traités à la DHA après leur passage dans l'estomac.

Pour cette évaluation, trois séries de digestion sont alors réalisées en parallèle :
- une première série avec deux enzymes (protocole AC), i.e. α-amylase et cellulase, afin d'estimer l'efficacité de la réaction de tannage (protéines résistantes aux enzymes du rumen),
- une deuxième série avec les trois enzymes (protocole ACP), i.e. α-amylase, cellulase et pepsine,
- une troisième série avec les quatre enzymes (protocole ACPT), i.e. α-amylase, cellulase, pepsine et trypsine.

La digestion enzymatique des protéines tannées ou non s'effectue en suivant le protocole de l'exemple 2 jusqu'à l'étape n°8.

L'étape n°9 et les suivantes sont alors :
9) on place au bain-marie à 40°C et agite continuellement pendant 18 h,
10) on ajuste au pH 7,5 avec de la soude 4 N,
11) on ajoute 500 µl de Trypsine à 5 g/l (activité spécifique de 10.000 U/mg de protéines),
12) on agite pendant 6 heures,
13) on recueille le digestat, laisse décanter, prélève deux fois 15 ml du surnageant et centrifuge à 4000 rpm pendant 20 min,
14) on récupère le surnageant et peut le congeler pour stockage.

La détermination du degré d'hydrolyse des protéines est réalisée également par la méthode de dosage des groupements aminés primaires des protéines traitées avec l'acide trinitrobenzène sulfonique (TNBS) de l'exemple 2.Les résultats de digestion enzymatique sur les échantillons sont exprimés en concentration en équivalent leucine (EL) exprimée en mmol/l.

Le tableau V suivant présente les valeurs de digestibilité obtenues pour les tourteaux de soja traités avec des doses variables en DHA (1 à 10 %) suivant la mise en oeuvre décrite dans l'exemple 1.

**Tableau V**

| Concentration en DHA | Sans traitement enzymatique | Protocole AC | Protocole ACP | Protocole ACPT |
|---|---|---|---|---|
| 0 | 2,78 | 3,08 | 14,42 | 30,45 |
| 1 % | 1,48 | 1,84 | 13,37 | 25,01 |
| 2,5 % | 0, 46 | 0,80 | 9,10 | 16,22 |
| 5 % | 0,48 | 0,71 | 8,05 | 14,08 |
| 10 % | 0 | 0,09 | 6,61 | 9,61 |

Des résultats équivalents sont obtenus avec les tourteaux de colza, comme l'indique le tableau VI suivant.

**Tableau VI**

| Concentration en DHA | Sans traitement enzymatique | Protocole AC | Protocole ACP | Protocole ACPT |
|---|---|---|---|---|
| 0 | 1,78 | 2,13 | 7,61 | 16,06 |
| 1 % | 1,43 | 1,80 | 6,52 | 12,83 |
| 2,5 % | 0,90 | 1,26 | 5,12 | 9,25 |
| 5 % | 0,56 | 0,89 | 4,19 | 7,10 |
| 10 % | 0,23 | 0,40 | 3,61 | 5,21 |

On constate que la digestion amylasique et cellulasique (protocole AC) a peu d'effet sur la libération des fonctions amines des protéines de colza et de soja. Cette digestion ne permet donc pas de restituer les protéines tannées à la DHA. Ces protéines resteront alors inaccessibles dans le rumen (l'activité cellulasique est importante dans le rumen).

Les digestions pepsique et trypsique des protéines libèrent efficacement des fonctions amines supplémentaires. La réaction de tannage à la DHA des protéines de soja et de colza est donc une réaction réversible, assurant une biodisponibilité des dites protéines dans l'estomac et l'intestin grêle.

Comme il l'avait été constaté dans l'exemple 2, les protéines de soja semblent plus facilement digérées par les complexes enzymatiques utilisés, que les protéines de colza.

Les résultats obtenus montrent enfin qu'au-delà de 5 % de tannage avec la DHA, l'efficacité de la réaction n'est plus aussi importante.

Le compromis entre tannage et digestion pepsique et trypsique se situe entre 1 et 2,5 % de DHA.

### Exemple 4

On évalue l'effet réticulant de la DHA vis-à-vis de la caséine de la manière suivante.

On prépare tout d'abord des colles mères de caséine et on utilise ensuite lesdites colles pour la préparation de films par « casting ».

### 1) procédé de préparation des colles de caséine

On prépare une solution aqueuse à 15 % de matière sèche de caséine H commercialisée par la société SVPC.

La solubilisation totale est obtenue par ajustement du pH à 9 par ajout de soude à 200 g/l, chauffage à 50°C et agitation à 1000 tours par minute pendant 20 min.

On refroidit cette solution à température ambiante et on incorpore ou non de la DHA sous forme d'une solution à 50 % de matière sèche, en ajustant le pH à 7,5 ou à 9 (DHA de pureté de 97 %, telle que commercialisée par la société SIGMA).

Les résultats des mesures de viscosité BROOKFIELD, en fonction du pH, sur les colles de caséine traitées ou non à la DHA (5 % d'une solution à 50 %, soit 2,5 % en sec par rapport à la caséine brute) sont présentés dans les tableaux suivants.

Le tableau VII présente l'évolution de la viscosité des colles en fonction du pH, sans DHA.

Le tableau VIII présente l'évolution de la viscosité des colles à pH 7,5 ou à pH 9 avec 2,5 % de DHA.

**Tableau VII**

| | | | | |
|---|---|---|---|---|
| PH | 7 | 7,5 | 8 | 9 |
| Viscosité BROOKFIELD à 40°C (mPa.s) | 6000 | 4500 | 5200 | 5000 |

**Tableau VIII.**

| Temps | 15 min | 30 min | 1 h | 2 h | 3 h | 12 h |
|---|---|---|---|---|---|---|
| Viscosité BROOKFIELD à 40°C (mPa.s) Mesurée à pH 7,5 | 6500 | Nd* | Nd | Nd | 25000 | Nd |
| Viscosité BROOKFIELD à 40°C (mPa.s) Mesurée à pH 9 | 6500 | 7500 | 13000 | 150000 | Nd | > 200000 |

| | | | | | | |
|---|---|---|---|---|---|---|
| *: Non déterminée | | | | | | |

L'addition de 5 % de DHA à 50 % de matière sèche (2,5 % en sec/caséine) se traduit, à un pH de 9, par une augmentation très nette mais progressive de la viscosité de la colle.

A pH de 7,5, la prise de viscosité est plus lente, mais notable.

Par comparaison, l'addition d'une quantité équivalente de formol s'est traduite par une prise en masse quasi-instantanée de la colle.

Ces essais démontrent un effet de réticulation progressive au cours du temps de la caséine par la DHA, à température ambiante, et cela de façon plus rapide à pH 9 qu'à pH 7,5.

### 2) procédé de préparation des films de caséine obtenus par casting

Les films de caséine sont préparés par « casting » (étalement sur support) à l'aide d'un appareillage SHEEN 1102 réglé à 700µm d'épaisseur, puis séchage lent en 24 h à 20°C et à 65 % d'humidité résiduelle.

Les caractéristiques des films obtenus sont présentées dans le tableau IX suivant.

**Tableau IX**

| | Aspect des films sans DHA | Aspect des films avec DHA |
|---|---|---|
| pH 7,5 | Films très fragiles, solubles dans l'eau en quelques minutes | Films fragmentés et fragiles insolubles dans l'eau |
| pH 9 | Films très fragiles, solubles dans l'eau en quelques minutes | Résistants et flexibles insolubles dans l'eau |

A pH 7,5 ou 9, avec 2,5 % de DHA en sec/caséine, la tenue à l'eau des films est particulièrement bonne, car les films restent intacts dans l'eau après plus d'une semaine, en présence d'un agent biocide. L'ajout de DHA confère ainsi une excellente tenue à l'eau tout en améliorant les propriétés mécaniques des films.

A ce titre, il convient de dire qu'il est impossible de fabriquer de tels films avec addition de formol, en raison d'une trop forte augmentation instantanée de la viscosité des colles.

### Exemple 5

L'effet insolubilisant de la DHA sur la caséine est estimé par une méthode de détermination de la teneur en matières solubles développée par la société Demanderesse.

Elle consiste à introduire 200 ml d'eau déminéralisée dans un bécher de 400 ml, y placer, sous agitation magnétique 5 g exactement de l'échantillon à analyser.

On homogénéise pendant 15 minutes à l'aide de l'agitateur magnétique et on centrifuge ensuite pendant 15 minutes à 5500 tours/minute. On prélève 25 ml du liquide surnageant et les introduit dans un cristallisoir taré.

On place ledit cristallisoir dans un four à micro-ondes pendant 10 minutes à 600 watts et 5 minutes à 900 watts jusqu'à déshydratation complète de l'échantillon. On le place ensuite dans un dessicateur pour le refroidir à température ambiante. On repèse le cristallisoir et note la masse m de résidu.

La teneur en matières solubles (Tms), exprimée en pourcentage en poids du produit, est donnée par la formule suivante : Tms = (m x 200 x 100)/ (25 x 5).

Le tableau X suivant présente les résultats de solubilités de films de caséine préparés par « casting » à température ambiante, à partir de solution de caséine à 15 % de matière sèche, selon les conditions données dans l'exemple 3.

**Tableau X**

| Dose de DHA en sec/caséine (%) | Tms (%) |
|---|---|
| 0 | 86 |
| 0,5 | 86 |
| 1,5 | 13,9 |
| 2,5 | 6,7 |

La solubilité aqueuse des films de caséine est abaissée par l'utilisation de DHA en quantité supérieure à 0,5 % en sec/caséine. La dose de 2,5% est préférée pour obtenir un film quasiment insoluble dans l'eau.

### Exemple 6

On réalise des essais d'enrobage de comprimés de mannitol (commercialisé par la société Demanderesse sous le nom de marque PEARLITOL^{®} DC) par des solutions aqueuses d'isolats de protéines de pois (commercialisés sous le nom de marque PISANE^{®} HD par la société COSUCRA).

On prépare trois solutions aqueuses de protéines de viscosité voisine de 500 mPa.s à température ambiante (mesurée sur viscosimètre BROOKFIELD):
- la première, en dispersant 12 g sec de PISANE® HD pour 100 g d'eau à 50°C et en ajustant en final le pH à 9,7 (solution A),
- la seconde, en dispersant 12 g sec de PISANE® HD et 1,2 g de solution aqueuse de DHA à 50 % de matière sèche, pour 100 g d'eau à 50°C et en ajustant en final le pH à 9,7 (solution B),
- la troisième, en dispersant 12 g sec de PISANE® HD, 1,2 g de solution aqueuse de DHA à 50 % de matière sèche, et 2,4 g de glycérol pour 100 g d'eau à 50°C et en ajustant en final le pH à 9,7(solution C).

Chacune de ces solutions est utilisée pour procéder à l'enrobage par pulvérisation de 200 g de comprimés de mannitol placés dans une turbine d'enrobage ERWEKA, tournant à une vitesse de 30 tours/minute, dans laquelle on introduit constamment de l'air sec pulsé à une température de 65°C.

Le débit de la solution est tel que l'enrobage est effectué en 45 minutes. L'enrobage protéique final sec représente alors environ 9 % de la masse initiale des comprimés.

On évalue la vitesse de fuite du mannitol au travers de l'enrobage protéique en plaçant, sous agitation mécanique, 7,5 g de comprimés dans 45,5 g d'eau déminéralisée, en mesurant la matière sèche (exprimée en brix) apparaissant dans l'eau au cours du temps.

Les résultats obtenus sont présentés dans le tableau XI suivant.

Tableau XI.

| Temps (min) | Comprimés de PEARLITOL^{®} DC non enrobés | Comprimés de PEARLITOL^{®} DC enrobés avec la solution A | Comprimés de PEARLITOL^{®} DC enrobés avec la solution B | Comprimés de PEARLITOL^{®} DC enrobés avec la solution C |
|---|---|---|---|---|
| 1 | 2,4 | 0,4 | 0,2 | 0,0 |
| 2 | 5,0 | 1,4 | 0,8 | 0,1 |
| 5 | 9,0 | 6,0 | 3,0 | 0,4 |
| 10 | 13,4 | 9,8 | 5,4 | 1,0 |
| 15 | 14,2 | 12,0 | 7,0 | 1,6 |
| 25 | 14,5 | 13,6 | 9,0 | 2,9 |
| 45 | 14,5 | 13,8 | 10,9 | 4,8 |
| 120 | 14,5 | 14,0 | 12,0 | 9,2 |
| 240 | 14,5 | 14,5 | 14,0 | 14,0 |

On constate que la fuite de mannitol par dissolution des comprimés est semblable entre les comprimés non enrobés et les comprimés enrobés avec la solution de protéines de pois PISANE^{®} HD sans DHA.

En revanche, les comprimés enrobés avec les solutions B ou C comprenant de la DHA présentent une fuite en mannitol nettement inférieure, notamment lorsque l'enrobage contient de la DHA en combinaison avec du glycérol.

Ces solutions B et C peuvent convenir à la préparation d'enrobage de produits alimentaires, de produits phytosanitaires (à base de pesticides, d'insecticides, d'herbicides, de fongicides...), de produits vétérinaires, de produits pharmaceutiques ou encore de produits industriels (à base d'enzymes, de colorants, de biocides...) pour obtenir des formes retard, des formulations protégées contre le vieillissement (UV, oxydation, température, hydrolyse...) ou des compositions présentant un aspect organoleptique amélioré (brillance, couleur...).

### Exemple 7

On estime les capacités « tannantes » du ribulose et du xylulose par le suivi de la réactivité de ces deux cétoses avec l'un des acides aminés les plus réactifs des protéines destinées à l'alimentation des ruminants : la lysine, en comparaison à l'acide glutamique.

L'action tannante est normalement obtenue par la liaison de l'aldéhyde avec le groupe ε-aminé de la lysine. On a alors la création d'une base de SCHIFF qui donne une couleur jaune aisément détectable.

On réalise la mesure de la coloration du milieu réactionnel au cours du temps (mesure de la coloration ICUMSA) de la manière suivante :
- on prépare 10 ml de chaque solution mère 1 M de ribulose, de xylulose, de lysine et d'acide glutamique dans de l'eau déminéralisée, à un pH de 6,
- on ajoute dans un tube à essais 5 ml de l'acide aminé et 5 ml du cétose à tester,
- on homogénéise le mélange obtenu et le place à réagir dans un bain d'huile à 80°C,
- on arrête la réaction à + 4°C.

Le tableau XII suivant présente la cinétique de condensation du ribulose et du xylulose avec la lysine et l'acide glutamique, en exprimant les résultats par l'absorbance à 420 nm, traduisant la coloration obtenue.

En tant que produits témoins, on réalise en parallèle des essais comparatifs de condensation de la lysine avec le fructose, et des essais de condensation du ribulose et du xylulose avec un acide aminé témoin, l'acide glutamique.

**Tableau XII**

| | | Temps (min) | | | | |
|---|---|---|---|---|---|---|
| | | 0 | 15 | 30 | 60 | 120 |
| Ribulose | Glu | 0,033 | 0,170 | 0,495 | 1,252 | 10,535 |
| | Lys | 0,086 | 1,743 | 4,653 | 15,899 | 38,658 |
| Xylulose | Glu | 0,096 | 0,666 | 0,922 | 1,930 | 3,021 |
| | Lys | 0,145 | 2,787 | 4,324 | 8,399 | 17,541 |
| Fructose | Glu | 0,008 | 0,021 | 0,044 | 0,112 | 0,280 |
| | Lys | 0,012 | 0,019 | 0,030 | 0,120 | 0,519 |

Il apparaît que le ribulose et le xylulose sont aussi de bons agents tannants pour la protection des protéines.

Par ailleurs, il est à noter que la cinétique de complexation sur acide aminé libre est assez rapide, car de premiers produits de condensation apparaissant dans les 15 premières minutes de la réaction.

Il apparaît également que le fructose est un mauvais agent tannant et ne convient absolument pas aux domaines d'application visés par la présente invention.

Les cétoses à 5 atomes de carbone sont donc suffisamment réactifs pour envisager un tannage efficace des protéines destinées à l'alimentation des animaux, à la préparation de compositions filmogènes, ou encore de matériaux biodégradables ou renouvelables.

### Exemple 8

On réalise des essais de fabrication d'aliments pour crevettes avec un tannage de protéines à l'aide de xylulose ou de DHA en lieu et place d'un urée-formol classique (composé MAXIBOND commercialisé par la société AGRESEARCH Inc, Joliet, IL, USA)

Le tableau XIII suivant présente la teneur en ingrédients (exprimée en % poids/poids) des 5 aliments ainsi préparés (pourcentage calculé pour un poids total des ingrédients de 3 kgs).

**Tableau XIII**

| Nature des ingrédients des aliments | Aliment N°1 | Aliment N°2 | Aliment N°3 | Aliment N°4 | Aliment N°5 |
|---|---|---|---|---|---|
| Farine de blé | 29,6 | 26,4 | 26,4 | 26,4 | 26,4 |
| Gluten | | 3,2 | 3,2 | 3,2 | 3,2 |
| Farine de poisson | 33,6 | 33,6 | 33,6 | 33,6 | 35,2 |
| Tourteaux de soja broyés | 12 | 12 | 12 | 12 | 12 |
| Huile de soja | 2,4 | 2,4 | 2,4 | 2,4 | 2,4 |
| Lécithine | 0,8 | 0,8 | 0,8 | 0,8 | 0,8 |
| MAXIBOND | 1,6 | 1,6 | | | |
| Xylulose (58 % MS) | | | 1,6 | | |
| DHA | | | | 1, 6 | |
| Eau | 20 | 20 | 20 | 20 | 20 |

On note ainsi que l'ALIMENT N°2 est tanné au MAXIBOND, l'ALIMENT N°3 est tanné au xylulose, l'ALIMENT N°4 est tanné à la DHA et l'ALIMENT N°5 est un aliment témoin non tanné.

L'ALIMENT N°1 est un témoin tanné au MAXIMBOND qui ne renferme pas de gluten.

L'aliment est préparé selon le mode opératoire suivant :
1) on mélange les ingrédients sous forme poudre (i.e. farine de blé, gluten, farine de poisson, tourteaux de soja broyés et MAXIBOND selon les formules) au mélangeur planétaire HOBART pendant 3 min à la vitesse 1,
2) on ajoute l'huile de soja et la lécithine,
3) on homogénéise au mélangeur planétaire HOBART 3 min à la vitesse 1,
4) on place le mélange dans une étuve à 95°C jusqu'à ce que la température du mélange atteigne 80°C,
5) dissout le xylulose ou la DHA dans de l'eau à 80°C,
6) on ajoute le xylulose ou la DHA ainsi solubilisée dans les proportions souhaitées,
7) on mélange au HOBART 5 min à la vitesse 1,
8) on texture à froid le mélange ainsi obtenu sur une extrudeuse monovis BUHLER dans les conditions présentées dans le tableau XIV ci-dessous,
9) on sèche ensuite les extrudats en étuve à 80°C pendant 15 heures environ.

**Tableau XIV**

| | Aliment N°1 | Aliment N°2 | Aliment N°3 | Aliment N°4 | Aliment N°5 |
|---|---|---|---|---|---|
| Humidité du mélange au départ (%) | 12,42 | 11,71 | 11,41 | 11,52 | 11,24 |
| Humidité après étuvage (%) | 8,54 | 9,59 | Nd* | 7,92 | Nd |
| Humidité avant extrusion (%) | 23,39 | 25,77 | 24,8 | 25,17 | 23,84 |

Les caractéristiques des compositions, puis des extrudats ainsi fabriqués sont présentés dans le tableau XV suivant.

**Tableau XV**

| | Aliment N°1 | Aliment N°2 | Aliment N°3 | Aliment N°4 | Aliment N°5 |
|---|---|---|---|---|---|
| Humidité après extrusion (%) | 20,66 | 22,53 | 22,35 | 23,49 | 23,02 |
| Humidité finale (%) | 6,46 | 7,43 | 6,22 | 7,71 | 7,52 |
| Temps de délitement dans de l'eau salée à 3,5 % (min) | > 120 | > 120 | > 120 | > 120 | < 10 |

| | | | | | |
|---|---|---|---|---|---|
| * : Nd = non déterminé | | | | | |

Comme il l'était attendu, l'aliment 5 non tanné n'est pas stable ; il se délite rapidement dans l'eau de mer

Les aliments tannés par le xylulose ou par la DHA montrent par contre de remarquables propriétés de stabilité, comparables aux aliments tannés à l'urée-formol.

La société Demanderesse a par ailleurs observé que la tenue à l'eau des ALIMENTS n°1 et n°4 sont équivalentes.

Il s'en déduit que la DHA ou le xylulose peuvent substituer avantageusement les agents de tannage plus toxiques comme l'urée-formol pour cette application particulière en aquaculture.

## Revendications

1. Procédé de réticulation des protéines selon lequel l'agent de réticulation est un cétose de 3 à 5 atomes de carbone et lesdites protéines sont choisies dans le groupe comprenant
- les protéines de céréales, comme notamment les protéines de maïs, de blé, de riz ;
- les protéines de protéagineux, comme notamment les protéines de pois, de luzerne, de lupin, d'orge, de millet, de sorgho ;
- les protéines d'oléagineux, comme notamment les protéines du soja, tels les tourteaux de soja, du colza, de lins, tels les tourteaux de colza, du tournesol, des arachides, du coton ; et
- les protéines de tubercules comme notamment de la pomme de terre, du manioc.

2. Procédé de réticulation des protéines selon la revendication 1, **caractérisé par le fait qu'**il consiste à :
1) mettre en contact les protéines et au moins un cétose de 3 à 5 atomes de carbone, à une concentration en poids sec du cétose de 0.5 à 20 %, de préférence de 1 à 10 % et plus préférentiellement de 1 à 5 % par rapport au poids sec des protéines brutes à traiter,
2) maintenir le contact entre les protéines et le cétose pendant une durée supérieure à quelques minutes, de préférence comprise entre 10 minutes à 60 minutes, de préférence pendant 30 minutes, à une température comprise entre 20 et 105°C, de préférence comprise entre 35 et 105°C,
3) et récupérer les protéines réticulées ainsi obtenues.

3. Procédé selon l'une quelconque des revendications 1 à 2, **caractérisé par le fait que** le cétose de 3 à 5 atomes de carbone est choisi dans le groupe constitué par la dihydroxyacétone, l'érythrulose, le ribulose et le xylulose, et est préférentiellement la dihydroxyacétone.

4. Protéines réticulées par des cétoses de 3 à 5 atomes de carbone susceptibles d'être obtenues selon le procédé de l'une quelconque des revendications 1 à 3.

5. Produit d'alimentation animale comprenant des protéines réticulées selon la revendication 4.

6. Produit d'alimentation animale selon la revendication 5, lesdites protéines pouvant en outre être choisies dans le groupe comprenant les protéines de tissus animaux, du lait, du sang, comme notamment la caséine, la gélatine, le collagène.

7. Produit d'alimentation animale selon l'une quelconque des revendications 5-6, destiné à l'alimentation de ruminants ou d'animaux de compagnie, ou destiné à l'aquaculture.

8. Procédé de préparation d'un produit d'alimentation animale, comprenant une étape de réticulation suivant le procédé de réticulation selon l'une quelconque des revendications 1-3, lesdites protéines pouvant en outre être choisies dans le groupe comprenant les protéines de tissus animaux, du lait, du sang, comme notamment la caséine, la gélatine, le collagène.

9. Utilisation de protéines réticulées selon la revendication 4, pour l'alimentation animale, notamment des ruminants ou des animaux de compagnie, ou en aquaculture.

10. Utilisation selon la revendication 9, **caractérisée par le fait que** les protéines réticulées y substituent les protéines traitées par des tannins minéraux, comme notamment les protéines tannées avec des sels de zinc.

11. Utilisation selon la revendication 9, **caractérisée par le fait que** les protéines réticulées y substituent les protéines traitées par des tannins aldéhydiques, comme notamment les protéines tannées au formaldéhyde, au glyoxal ou au glutaraldéhyde, et plus particulièrement les protéines tannées au formol.

12. Procédé de préparation d'une composition filmogène pour revêtements, enrobages ou adhésifs,
comprenant une réticulation de protéines selon laquelle l'agent de réticulation est un cétose de 3 à 5 atomes de carbone et lesdites protéines sont choisies dans le groupe comprenant
- les protéines de tissus animaux, du lait, du sang, comme notamment la caséine, la gélatine, le collagène ;
- les protéines de céréales, comme notamment les protéines de maïs, de blé, de riz ;
- les protéines de protéagineux, comme notamment les protéines de pois, de luzerne, de lupin, d'orge, de millet, de sorgho ;
- les protéines d'oléagineux, comme notamment les protéines du soja, tels les tourteaux de soja, du colza, de lins, tels les tourteaux de colza, du tournesol, des arachides, du coton ; et
- les protéines de tubercules comme notamment de la pomme de terre, du manioc.

13. Procédé de préparation d'une composition filmogène pour revêtements, enrobages ou adhésifs selon la revendication 12, **caractérisé par le fait que** la réticulation consiste à :
1) mettre en contact les protéines et au moins un cétose de 3 à 5 atomes de carbone, à une concentration en poids sec du cétose de 0.5 à 20 %, de préférence de 1 à 10 % et plus préférentiellement de 1 à 5 % par rapport au poids sec des protéines brutes à traiter,
2) maintenir le contact entre les protéines et le cétose pendant une durée supérieure à quelques minutes, de préférence comprise entre 10 minutes à 60 minutes, de préférence pendant 30 minutes, à une température comprise entre 20 et 105°C, de préférence comprise entre 35 et 105°C,
3) et récupérer les protéines réticulées ainsi obtenues.

14. Procédé selon l'une quelconque des revendications 12-13, **caractérisé par le fait que** le cétose de 3 à 5 atomes de carbone est choisi dans le groupe constitué par la dihydroxyacétone, l'érythrulose, le ribulose et le xylulose, et est préférentiellement la dihydroxyacétone.

15. Procédé selon l'une quelconque des revendications 12-14, **caractérisé par le fait que** les protéines réticulées y substituent les protéines tannées par des tannins synthétiques, comme notamment les protéines tannées avec des résines épichlorhydrines, les résines polyamide-amine-épichlorhydrines, les résines polyéthylène-imines ou les résines isocyanates.

16. Procédé selon l'une quelconque des revendications 12-14, **caractérisé par le fait que** les protéines réticulées y substituent les protéines tannées par des tannins aldéhydiques, comme notamment les protéines tannées au formaldéhyde, au glyoxal ou au glutaraldéhyde, et plus particulièrement les protéines tannées au formol.

17. Utilisation, pour la préparation d'une composition filmogène pour revêtements, enrobages ou adhésifs, de protéines réticulées susceptibles d'être obtenues par un procédé de réticulation des protéines selon lequel l'agent de réticulation est un cétose de 3 à 5 atomes de carbone et lesdites protéines sont choisies dans le groupe comprenant
- les protéines de tissus animaux, du lait, du sang, comme notamment la caséine, la gélatine, le collagène;
- les protéines de céréales, comme notamment les protéines de maïs, de blé, de riz ;
- les protéines de protéagineux, comme notamment les protéines de pois, de luzerne, de lupin, d'orge, de millet, de sorgho ;
- les protéines d'oléagineux, comme notamment les protéines du soja, tels les tourteaux de soja, du colza, de lins, tels les tourteaux de colza, du tournesol, des arachides, du coton ; et les protéines de tubercules comme notamment de la pomme de terre, du manioc.

18. Utilisation selon la revendication 17, **caractérisée par le fait que** le procédé de réticulation consiste à :
1) mettre en contact les protéines et au moins un cétose de 3 à 5 atomes de carbone, à une concentration en poids sec du cétose de 0.5 à 20 %, de préférence de 1 à 10 % et plus préférentiellement de 1 à 5 % par rapport au poids sec des protéines brutes à traiter,
2) maintenir le contact entre les protéines et le cétose pendant une durée supérieure à quelques minutes, de préférence comprise entre 10 minutes à 60 minutes, de préférence pendant 30 minutes, à une température comprise entre 20 et 105°C, de préférence comprise entre 35 et 105°C,
3) et récupérer les protéines réticulées ainsi obtenues.

19. Utilisation selon l'une quelconque des revendications 17-18, **caractérisée par le fait que** le cétose de 3 à 5 atomes de carbone est choisi dans le groupe constitué par la dihydroxyacétone, l'érythrulose, le ribulose et le xylulose, et est préférentiellement la dihydroxyacétone.

20. Utilisation selon l'une quelconque des revendications 17-19, **caractérisée par le fait que** les protéines réticulées y substituent les protéines tannées par des tannins synthétiques, comme notamment les protéines tannées avec des résines épichlorhydrines, les résines polyamide-amine-épichlorhydrines, les résines polyéthylène-imines ou les résines isocyanates.

21. Utilisation selon l'une quelconque des revendications 17-19, **caractérisée par le fait que** les protéines réticulées y substituent les protéines tannées par des tannins aldéhydiques, comme notamment les protéines tannées au formaldéhyde, au glyoxal ou au glutaraldéhyde, et plus particulièrement les protéines tannées au formol.

22. Procédé de préparation d'un matériau protéique biodégradable ou renouvelable,
comprenant une réticulation de protéines selon laquelle l'agent de réticulation est un cétose de 3 à 5 atomes de carbone et lesdites protéines sont choisies dans le groupe comprenant
- les protéines de tissus animaux, du lait, du sang, comme notamment la caséine, la gélatine, le collagène ;
- les protéines de céréales, comme notamment les protéines de maïs, de blé, de riz ;
- les protéines de protéagineux, comme notamment les protéines de pois, de luzerne, de lupin, d'orge, de millet, de sorgho ;
- les protéines d'oléagineux, comme notamment les protéines du soja, tels les tourteaux de soja, du colza, de lins, tels les tourteaux de colza, du tournesol, des arachides, du coton ; et
- les protéines de tubercules comme notamment de la pomme de terre, du manioc.

23. Procédé préparation de matériaux protéiques biodégradables ou renouvelables selon la revendication 22, **caractérisé par le fait que** la réticulation consiste à :
1) mettre en contact les protéines et au moins un cétose de 3 à 5 atomes de carbone, à une concentration en poids sec du cétose de 0.5 à 20 %, de préférence de 1 à 10 % et plus préférentiellement de 1 à 5 % par rapport au poids sec des protéines brutes à traiter,
2) maintenir le contact entre les protéines et le cétose pendant une durée supérieure à quelques minutes, de préférence comprise entre 10 minutes à 60 minutes, de préférence pendant 30 minutes, à une température comprise entre 20 et 105°C, de préférence comprise entre 35 et 105°C,
3) et récupérer les protéines réticulées ainsi obtenues.

24. Procédé selon l'une quelconque des revendications 22-23, **caractérisé par le fait que** le cétose de 3 à 5 atomes de carbone est choisi dans le groupe constitué par la dihydroxyacétone, l'érythrulose, le ribulose et le xylulose, et est préférentiellement la dihydroxyacétone.

25. Procédé selon l'une quelconque des revendications 22-24, **caractérisé par le fait que** les protéines réticulées y substituent les protéines tannées par des tannins minéraux, comme notamment les protéines tannées au chrome.

26. Procédé selon l'une quelconque des revendications 22-24, **caractérisé par le fait que** les protéines réticulées y substituent les protéines tannées par des tannins aldéhydiques, comme notamment les protéines tannées au formaldéhyde, au glyoxal ou au glutaraldéhyde, et plus particulièrement les protéines tannées au formol.

27. Procédé selon l'une quelconque des revendications 22-26, **caractérisé par le fait que** ledit matériau protéique biodégradable ou renouvelable est choisi parmi les films plastiques, les cuirs, les fibres textiles, les gélules, les capsules, ou les compositions protéiques alimentaires.

28. Utilisation, pour la préparation de matériaux protéiques biodégradables ou renouvelables, de protéines réticulées susceptibles d'être obtenues par un procédé de réticulation des protéines selon lequel l'agent de réticulation est un cétose de 3 à 5 atomes de carbone et lesdites protéines sont choisies dans le groupe comprenant
- les protéines de tissus animaux, du lait, du sang, comme notamment la caséine, la gélatine, le collagène ;
- les protéines de céréales, comme notamment les protéines de maïs, de blé, de riz ;
- les protéines de protéagineux, comme notamment les protéines de pois, de luzerne, de lupin, d'orge, de millet, de sorgho ;
- les protéines d'oléagineux, comme notamment les protéines du soja, tels les tourteaux de soja, du colza, de lins, tels les tourteaux de colza, du tournesol, des arachides, du coton ; et les protéines de tubercules comme notamment de la pomme de terre, du manioc.

29. Utilisation selon la revendication 28, **caractérisée par le fait que** le procédé de réticulation consiste à :
1) mettre en contact les protéines et au moins un cétose de 3 à 5 atomes de carbone, à une concentration en poids sec du cétose de 0.5 à 20 %, de préférence de 1 à 10 % et plus préférentiellement de 1 à 5 % par rapport au poids sec des protéines brutes à traiter,
2) maintenir le contact entre les protéines et le cétose pendant une durée supérieure à quelques minutes, de préférence comprise entre 10 minutes à 60 minutes, de préférence pendant 30 minutes, à une température comprise entre 20 et 105°C, de préférence comprise entre 35 et 105°C,
3) et récupérer les protéines réticulées ainsi obtenues.

30. Utilisation selon l'une quelconque des revendications 28-29, **caractérisée par le fait que** le cétose de 3 à 5 atomes de carbone est choisi dans le groupe constitué par la dihydroxyacétone, l'érythrulose, le ribulose et le xylulose, et est préférentiellement la dihydroxyacétone.

31. Utilisation selon l'une quelconque des revendications 28-30, **caractérisée par le fait que** les protéines réticulées y substituent les protéines tannées par des tannins minéraux, comme notamment les protéines tannées au chrome.

32. Utilisation selon l'une quelconque des revendications 28-30, **caractérisée par le fait que** les protéines réticulées y substituent les protéines tannées par des tannins aldéhydiques, comme notamment les protéines tannées au formaldéhyde, au glyoxal ou au glutaraldéhyde, et plus particulièrement les protéines tannées au formol.

33. Utilisation selon l'une quelconque des revendications 28-32, **caractérisée par le fait que** ledit matériau protéique biodégradable ou renouvelable est choisi parmi les films plastiques, les cuirs, les fibres textiles, les gélules, les capsules, ou les compositions protéiques alimentaires

## Claims

1. Process for cross-linking of proteins, according to which the cross-linking agent is a ketose with 3 to 5 carbon atoms and said proteins are chosen from the group comprising
- grain proteins, such as, in particular, maize proteins, wheat proteins, rice proteins;
- proteinaceous-seed proteins, such as, in particular, pea proteins, lucerne proteins, lupin proteins, barley proteins, millet proteins, sorghum proteins;
- oilseed proteins, such as, in particular, soybean proteins, for instance soybean cakes, rapeseed proteins, flax proteins, for instance rapeseed cakes, sunflower proteins, groundnut proteins, cotton proteins; and
- tuber proteins, such as, in particular, potato proteins, cassava proteins.

2. Process for cross-linking of proteins according to Claim 1, **characterised in that** it consists in:
1) bringing the proteins and at least one ketose with 3 to 5 carbon atoms into contact at a concentration of the ketose, by dry weight, from 0.5% to 20 %, preferably from 1% to 10 % and more preferentially from 1 % to 5%, relative to the dry weight of the crude proteins to be treated,
2) maintaining the contact between the proteins and the ketose for a period longer than a few minutes, preferably between 10 minutes and 60 minutes, preferably for 30 minutes, at a temperature between 20 °C and 105 °C, preferably between 35 °C and 105 °C,
3) and recovering the cross-linked proteins thus obtained.

3. Process according to any one of Claims 1 to 2, **characterised in that** the ketose with 3 to 5 carbon atoms is chosen from the group constituted by dihydroxyacetone, erythrulose, ribulose and xylulose, and is preferentially dihydroxyacetone.

4. Proteins cross-linked by ketoses with 3 to 5 carbon atoms, which are capable of being obtained in accordance with the process of any one of Claims 1 to 3.

5. Animal-feed product including cross-linked proteins according to Claim 4.

6. Animal-feed product according to Claim 5, said proteins being capable, in addition, of being chosen from the group comprising animal-tissue proteins, milk proteins, blood proteins, such as, in particular, casein, gelatin, collagen.

7. Animal-feed product according to any one of Claims 5-6, intended for the feeding of ruminants or pets, or intended for aquaculture.

8. Process for preparing an animal-feed product, including a cross-linking step in accordance with the cross-linking process according to any one of Claims 1-3, said proteins being capable, in addition, of being chosen from the group comprising animal-tissue proteins, milk proteins, blood proteins, such as, in particular, casein, gelatin, collagen.

9. Use of cross-linked proteins according to Claim 4 for animal feed, in particular for the feeding of ruminants or pets, or in aquaculture.

10. Use according to Claim 9, **characterised in that** the cross-linked proteins substitute for proteins treated by mineral tannins, such as, in particular, proteins tanned with zinc salts.

11. Use according to Claim 9, **characterised in that** the cross-linked proteins substitute for proteins treated by aldehyde tannins, such as, in particular, proteins tanned in formaldehyde, in glyoxal or in glutaraldehyde, and more particularly proteins tanned in formalin.

12. Process for preparing a film-forming composition for linings, coatings or adhesives, including a cross-linking of proteins, according to which the cross-linking agent is a ketose with 3 to 5 carbon atoms and said proteins are chosen from the group comprising
- animal-tissue proteins, milk proteins, blood proteins, such as, in particular, casein, gelatin, collagen;
- grain proteins, such as, in particular, maize proteins, wheat proteins, rice proteins;
- proteinaceous-seed proteins, such as, in particular, pea proteins, lucerne proteins, lupin proteins, barley proteins, millet proteins, sorghum proteins;
- oilseed proteins, such as, in particular, soybean proteins, for instance soybean cakes, rapeseed proteins, flax proteins, for instance rapeseed cakes, sunflower proteins, groundnut proteins, cotton proteins; and
- tuber proteins, such as, in particular, potato proteins, cassava proteins.

13. Process for preparing a film-forming composition for linings, coatings or adhesives according to Claim 12, **characterised in that** the cross-linking consists in:
1) bringing the proteins and at least one ketose with 3 to 5 carbon atoms into contact at a concentration of the ketose, by dry weight, from 0.5% to 20%, preferably from 1% to 10% and more preferentially from 1% to 5%, relative to the dry weight of the crude proteins to be treated,
2) maintaining the contact between the proteins and the ketose for a period longer than a few minutes, preferably between 10 minutes and 60 minutes, preferably for 30 minutes, at a temperature between 20 °C and 105 °C, preferably between 35 °C and 105 °C,
3) and recovering the cross-linked proteins thus obtained.

14. Process according to any one of Claims 12-13, **characterised in that** the ketose with 3 to 5 carbon atoms is chosen from the group constituted by dihydroxyacetone, erythrulose, ribulose and xylulose, and is preferentially dihydroxyacetone.

15. Process according to any one of Claims 12-14, **characterised in that** the cross-linked proteins substitute for proteins tanned by synthetic tannins, such as, in particular, proteins tanned with epichlorohydrin resins, polyamide-amine-epichlorohydrin resins, polyethylene-imine resins or isocyanate resins.

16. Process according to any one of Claims 12-14, **characterised in that** the cross-linked proteins substitute for proteins tanned by aldehyde tannins, such as, in particular, proteins tanned in formaldehyde, in glyoxal or in glutaraldehyde, and more particularly proteins tanned in formalin.

17. Use, for the preparation of a film-forming composition for linings, coatings or adhesives, of cross-linked proteins that are capable of being obtained by a process for cross-linking of proteins, according to which the cross-linking agent is a ketose with 3 to 5 carbon atoms and said proteins are chosen from the group comprising
- animal-tissue proteins, milk proteins, blood proteins, such as, in particular, casein, gelatin, collagen;
- grain proteins, such as, in particular, maize proteins, wheat proteins, rice proteins;
- proteinaceous-seed proteins, such as, in particular, pea proteins, lucerne proteins, lupin proteins, barley proteins, millet proteins, sorghum proteins;
- oilseed proteins, such as, in particular, soybean proteins, for instance soybean cakes, rapeseed proteins, flax proteins, for instance rapeseed cakes, sunflower proteins, groundnut proteins, cotton proteins; and tuber proteins, such as, in particular, potato proteins, cassava proteins.

18. Use according to Claim 17, **characterised in that** the cross-linking process consists in:
1) bringing the proteins and at least one ketose with 3 to 5 carbon atoms into contact at a concentration of the ketose, by dry weight, from 0.5 % to 20 %, preferably from 1 % to 10 % and more preferentially from 1 % to 5 %, relative to the dry weight of the crude proteins to be treated,
2) maintaining the contact between the proteins and the ketose for a period longer than a few minutes, preferably between 10 minutes and 60 minutes, preferably for 30 minutes, at a temperature between 20 °C and 105 °C, preferably between 35 °C and 105 °C,
3) and recovering the cross-linked proteins thus obtained.

19. Use according to any one of Claims 17-18, **characterised in that** the ketose with 3 to 5 carbon atoms is chosen from the group constituted by dihydroxyacetone, erythrulose, ribulose and xylulose, and is preferentially dihydroxyacetone.

20. Use according to any one of Claims 17-19, **characterised in that** the cross-linked proteins substitute for proteins tanned by synthetic tannins, such as, in particular, proteins tanned with epichlorohydrin resins, polyamide-amine-epichlorohydrin resins, polyethylene-imine resins or isocyanate resins.

21. Use according to any one of Claims 17-19, **characterised in that** the cross-linked proteins substitute for proteins tanned by aldehyde tannins, such as, in particular, proteins tanned in formaldehyde, in glyoxal or in glutaraldehyde, and more particularly proteins tanned in formalin.

22. Process for preparing a biodegradable or renewable proteinic material, including a cross-linking of proteins, according to which the cross-linking agent is a ketose with 3 to 5 carbon atoms and said proteins are chosen from the group comprising
- animal-tissue proteins, milk proteins, blood proteins, such as, in particular, casein, gelatin, collagen;
- grain proteins, such as, in particular, maize proteins, wheat proteins, rice proteins;
- proteinaceous-seed proteins, such as, in particular, pea proteins, lucerne proteins, lupin proteins, barley proteins, millet proteins, sorghum proteins;
- oilseed proteins, such as, in particular, soybean proteins, for instance soybean cakes, rapeseed proteins, flax proteins, for instance rapeseed cakes, sunflower proteins, groundnut proteins, cotton proteins; and
- tuber proteins, such as, in particular, potato proteins, cassava proteins.

23. Process for preparing biodegradable or renewable proteinic materials according to Claim 22, **characterised in that** the cross-linking consists in:
1) bringing the proteins and at least one ketose with 3 to 5 carbon atoms into contact at a concentration of the ketose, by dry weight, from 0.5% to 20%, preferably from 1% to 10% and more preferentially from 1% to 5%, relative to the dry weight of the crude proteins to be treated,
2) maintaining the contact between the proteins and the ketose for a period longer than a few minutes, preferably between 10 minutes and 60 minutes, preferably for 30 minutes, at a temperature between 20 °C and 105 °C, preferably between 35 °C and 105 °C,
3) and recovering the cross-linked proteins thus obtained.

24. Process according to any one of Claims 22-23, **characterised in that** the ketose with 3 to 5 carbon atoms is chosen from the group constituted by dihydroxyacetone, erythrulose, ribulose and xylulose, and is preferentially dihydroxyacetone.

25. Process according to any one of Claims 22-24, **characterised in that** the cross-linked proteins substitute for proteins tanned by mineral tannins, such as, in particular, proteins tanned in chromium.

26. Process according to any one of Claims 22-24, **characterised in that** the cross-linked proteins substitute for proteins tanned by aldehyde tannins, such as, in particular, proteins tanned in formaldehyde, in glyoxal or in glutaraldehyde, and more particularly proteins tanned in formalin.

27. Process according to any one of Claims 22-26, **characterised in that** said biodegradable or renewable proteinic material is chosen from plastic films, leathers, textile fibres, gel capsules, other capsules, or nutritional proteinic compositions.

28. Use, for the preparation of biodegradable or renewable proteinic materials, of cross-linked proteins that are capable of being obtained by a process for cross-linking of proteins, according to which the cross-linking agent is a ketose with 3 to 5 carbon atoms and said proteins are chosen from the group comprising
- animal-tissue proteins, milk proteins, blood proteins, such as, in particular, casein, gelatin, collagen;
- grain proteins, such as, in particular, maize proteins, wheat proteins, rice proteins;
- proteinaceous-seed proteins, such as, in particular, pea proteins, lucerne proteins, lupin proteins, barley proteins, millet proteins, sorghum proteins;
- oilseed proteins, such as, in particular, soybean proteins, for instance soybean cakes, rapeseed proteins, flax proteins, for instance rapeseed cakes, sunflower proteins, groundnut proteins, cotton proteins; and tuber proteins, such as, in particular, potato proteins, cassava proteins.

29. Use according to Claim 28, **characterised in that** the cross-linking process consists in:
1) bringing the proteins and at least one ketose with 3 to 5 carbon atoms into contact at a concentration of the ketose, by dry weight, from 0.5% to 20 %, preferably from 1% to 10 % and more preferentially from 1% to 5%, relative to the dry weight of the crude proteins to be treated,
2) maintaining the contact between the proteins and the ketose for a period longer than a few minutes, preferably between 10 minutes and 60 minutes, preferably for 30 minutes, at a temperature between 20 °C and 105 °C, preferably between 35 °C and 105 °C,
3) and recovering the cross-linked proteins thus obtained.

30. Use according to any one of Claims 28-29, **characterised in that** the ketose with 3 to 5 carbon atoms is chosen from the group constituted by dihydroxyacetone, erythrulose, ribulose and xylulose, and is preferentially dihydroxyacetone.

31. Use according to any one of Claims 28-30, **characterised in that** the cross-linked proteins substitute for proteins tanned by mineral tannins, such as, in particular, proteins tanned in chromium.

32. Use according to any one of Claims 28-30, **characterised in that** the cross-linked proteins substitute for proteins tanned by aldehyde tannins, such as, in particular, proteins tanned in formaldehyde, in glyoxal or in glutaraldehyde, and more particularly proteins tanned in formalin.

33. Use according to any one of Claims 28-32, **characterised in that** said biodegradable or renewable proteinic material is chosen from plastic films, leathers, textile fibres, hard capsules, other capsules, or nutritional proteinic compositions.

## Patentansprüche

1. Verfahren zur Vernetzung von Proteinen, gemäß welchen das Vernetzungsmittel eine Cetose von 3 bis 5 Kohlenstoffatomen ist und die Proteine aus der Gruppe ausgewählt sind, die umfasst:
- Getreideproteine, wie insbesondere Mais-, Weizen-, Reisproteine;
- Eiweißpflanzenproteine, wie insbesondere Proteine von Erbsen, Luzerne, Lupine, Gerste, Hirse, Sorgum;
- Ölsaatenproteine, wie insbesondere Proteine von Soja, wie Sojakuchen, Colza, Lein, wie Colzakuchen, Sonnenblume, Erdnuss, Baumwolle; und
- Proteine von Knollen, wie insbesondere Kartoffel, Maniok.

2. Verfahren zur Vernetzung von Proteinen nach Anspruch 1, **dadurch gekennzeichnet, dass** es darin besteht, dass:
1) die Proteine und mindestens eine Cetose von 3 bis 5 Kohlenstoffatomen mit einer Cetose-Konzentration in Trockengewicht von 0,5 bis 20 %, vorzugsweise 1 bis 10 % und noch bevorzugter 1 bis 5 %, bezogen auf das Trockengewicht der zu behandelnden Rohproteine, in Kontakt gebracht werden,
2) der Kontakt zwischen den Proteinen und der Cetose während einer Zeit von mehr als einigen Minuten, vorzugsweise zwischen 10 Minuten bis 60 Minuten, vorzugsweise während 30 Minute, bei einer Temperatur zwischen 20 und 105°C, vorzugsweise zwischen 35 und 105°C, aufrechterhalten wird,
3) und die auf diese Weise erhaltenen vernetzten Proteine gewonnen werden.

3. Verfahren nach einem der Ansprüche 1 bis 2, **dadurch gekennzeichnet, dass** die Cetose von 3 bis 5 Kohlenstoffatomen aus der Gruppe ausgewählt ist, die aus Dihydroxyaceton, Erythrulose, Ribulose und Xylulose besteht und vorzugsweise Dihydroxyaceton ist.

4. Durch Cetosen von 3 bis 5 Kohlenstoffatomen vernetzte Proteine, die gemäß dem Verfahren von einem der Ansprüche 1 bis 3 erhalten werden können.

5. Tiernahrungsprodukt, umfassend vernetzte Proteine gemäß Anspruch 4.

6. Tiernahrungsprodukt nach Anspruch 5, wobei die Proteine außerdem aus der Gruppe ausgewählt werden können, die die Proteine von tierischen Geweben, Milch, Blut, wie insbesondere Casein, Gelatine, Collagen, umfasst.

7. Tiernahrungsprodukt nach einem der Ansprüche 5 bis 6, das für die Ernährung von Wiederkäuern oder Haustieren bestimmt ist oder für die Aquakultur bestimmt ist.

8. Verfahren zur Herstellung eines Tiernahrungsprodukts, umfassend einen Schritt der Vernetzung gemäß dem Vernetzungsverfahren nach einem der Ansprüche 1 bis 3, wobei diese Proteine außerdem aus der Gruppe ausgewählt werden können, die die Proteine von tierischen Geweben, Milch, Blut, wie insbesondere Casein, Gelatine, Collagen umfasst.

9. Verwendung von vernetzten Proteinen nach Anspruch 4 für die Tierernährung, insbesondere von Wiederkäuern oder Haustieren, oder in der Aquakultur.

10. Verwendung nach Anspruch 9, **dadurch gekennzeichnet, dass** die vernetzten Proteine darin die mit mineralischen Tanninen behandelnden Proteine, wie insbesondere die mit Zinksalzen gegerbten Proteine, substituieren.

11. Verwendung nach Anspruch 9, **dadurch gekennzeichnet, dass** die vernetzten Proteine darin die mit aldehydischen Tanninen behandelten Proteine, wie insbesondere die mit Formaldehyd, mit Glyoxal oder mit Glutaraldehyd gegerbten Proteine, und insbesondere die mit Formol gegerbten Proteine, substituieren.

12. Verfahren zur Herstellung einer filmbildenden Zusammensetzung für Überzüge, Beschichtungen oder Klebstoffe,
umfassend eine Vernetzung von Proteinen, gemäß welcher das Vernetzungsmittel eine Cetose von 3 bis 5 Kohlenstoffatomen ist und die Proteine aus der Gruppe ausgewählt sind, die umfasst:
- Proteine von tierischen Geweben, Milch, Blut, wie insbesondere Casein, Gelatine, Collagen,
- Getreideproteine, wie insbesondere Mais-, Weizen-, Reisproteine;
- Eiweißpflanzenproteine, wie insbesondere Proteine von Erbsen, Luzerne, Lupine, Gerste, Hirse, Sorgum;
- Ölsaatenproteine, wie insbesondere Proteine von Soja, wie Sojakuchen, Colza, Lein, wie Colzakuchen, Sonnenblume, Erdnuss, Baumwolle; und
- Proteine von Knollen, wie insbesondere Kartoffel, Maniok.

13. Verfahren zur Herstellung einer filmbildenden Zusammensetzung für Überzüge, Beschichtungen oder Klebstoffe nach Anspruch 12, **dadurch gekennzeichnet, dass** die Vernetzung darin besteht, dass:
1) die Proteine und mindestens eine Cetose von 3 bis 5 Kohlenstoffatomen mit einer Cetose-Konzentration in Trockengewicht von 0,5 bis 20 %, vorzugsweise 1 bis 10 % und noch bevorzugter 1 bis 5 %, bezogen auf das Trockengewicht der zu behandelnden Rohproteine, in Kontakt gebracht werden,
2) der Kontakt zwischen den Proteinen und der Cetose während einer Zeit von mehr als einigen Minuten, vorzugsweise zwischen 10 Minuten bis 60 Minuten, vorzugsweise während 30 Minute, bei einer Temperatur zwischen 20 und 105°C, vorzugsweise zwischen 35 und 105°C, aufrechterhalten wird,
3) und die auf diese Weise erhaltenen vernetzten Proteine gewonnen werden.

14. Verfahren nach einem der Ansprüche 12 bis 13, **dadurch gekennzeichnet, dass** die Cetose mit 3 bis 5 Kohlenstoffatomen aus der Gruppe ausgewählt ist, die aus Dihydroxyaceton, Erythrulose, Ribulose und Xylulose besteht und vorzugsweise Dihydroxyaceton ist.

15. Verfahren nach einem der Ansprüche 12 bis 14, **dadurch gekennzeichnet, dass** die vernetzten Proteine darin die mit synthetischen Gerbstoffen behandelten Proteine, wie insbesondere die mit Epichlorhydrinharzen gerbstoffbehandelten Proteine, die Polyamid-aminoepichlorhydrin-Harze, die Polyethylen-imin-Harze oder die Isocyanatharze.

16. Verfahren nach einem der Ansprüche 12 bis 14, **dadurch gekennzeichnet, dass** die vernetzten Proteine darin die mit aldehydischen Gerbstoffen gerbstoffbehandelten Proteine substituieren, wie insbesondere die mit Formaldehyd, Glyoxal oder Glutaraldehyd gerbstoffbehandelten Proteine, und insbesondere die mit Formol gerbstoffbehandelten Proteine.

17. Verwendung, für die Herstellung einer filmbildenden Zusammensetzung für Überzüge, Beschichtungen oder Klebstoffe,
von vernetzten Proteinen, die durch ein Verfahren zur Vernetzung von Proteinen erhalten werden können, gemäß welchem das Vernetzungsmittel eine Cetose von 3 bis 5 Kohlenstoffatomen ist und die Proteine aus der Gruppe ausgewählt sind, die umfasst:
- Proteine von tierischen Geweben, Milch, Blut, wie insbesondere Casein, Gelatine, Collagen,
- Getreideproteine, wie insbesondere Mais-, Weizen-, Reisproteine;
- Eiweißpflanzenproteine, wie insbesondere Proteine von Erbsen, Luzerne, Lupine, Gerste, Hirse, Sorgum;
- Ölsaatenproteine, wie insbesondere Proteine von Soja, wie Sojakuchen, Colza, Lein, wie Colzakuchen, Sonnenblume, Erdnuss, Baumwolle; und
- Proteine von Knollen, wie insbesondere Kartoffel, Maniok.

18. Verwendung nach Anspruch 17, **dadurch gekennzeichnet, dass** das Vernetzungsverfahren darin besteht, dass:
1) die Proteine und mindestens eine Cetose von 3 bis 5 Kohlenstoffatomen mit einer Cetose-Konzentration in Trockengewicht von 0,5 bis 20 %, vorzugsweise 1 bis 10 % und noch bevorzugter 1 bis 5 %, bezogen auf das Trockengewicht der zu behandelnden Rohproteine, in Kontakt gebracht werden,
2) der Kontakt zwischen den Proteinen und der Cetose während einer Zeit von mehr als einigen Minuten, vorzugsweise zwischen 10 Minuten bis 60 Minuten, vorzugsweise während 30 Minute, bei einer Temperatur zwischen 20 und 105°C, vorzugsweise zwischen 35 und 105°C, aufrechterhalten wird,
3) und die auf diese Weise erhaltenen vernetzten Proteine gewonnen werden.

19. Verwendung nach einem der Ansprüche 17 bis 18, **dadurch gekennzeichnet, dass** die Cetose von 3 bis 5 Kohlenstoffatomen aus der Gruppe ausgewählt ist, die aus Dihydroxyaceton, Erythrulose, Ribulose und Xylulose besteht, und vorzugsweise Dihydroxyaceton ist.

20. Verwendung nach einem der Ansprüche 17 bis 19, **dadurch gekennzeichnet, dass** die vernetzten Proteine darin die mit synthetischen Gerbstoffen behandelten Proteine, wie insbesondere die mit Epichlorhydrinharzen gerbstoffbehandelten Proteine, die Polyamid-aminoepichlorhydrin-Harze, die Polyethylen-imin-Harze oder die Isocyanatharze.

21. Verwendung nach einem der Ansprüche 17 bis 19, **dadurch gekennzeichnet, dass** die vernetzten Proteine darin die mit aldehydischen Gerbstoffen gerbstoffbehandelten Proteine substituieren, wie insbesondere die mit Formaldehyd, Glyoxal oder Glutaraldehyd gerbstoffbehandelten Proteine, und insbesondere die mit Formol gerbstoffbehandelten Proteine.

22. Verfahren zur Herstellung eines biologisch abbaubauren oder erneuerbaren Proteinmaterials,
umfassend eine Vernetzung von Proteinen, gemäß welcher das Vernetzungsmittel eine Cetose von 3 bis 5 Kohlenstoffatomen ist und die Proteine aus der Gruppe ausgewählt sind, die umfasst:
- Proteine von tierischen Geweben, Milch, Blut, wie insbesondere Casein, Gelatine, Collagen,
- Getreideproteine, wie insbesondere Mais-, Weizen-, Reisproteine;
- Eiweißpflanzenproteine, wie insbesondere Proteine von Erbsen, Luzerne, Lupine, Gerste, Hirse, Sorgum;
- Ölsaatenproteine, wie insbesondere Proteine von Soja, wie Sojakuchen, Colza, Lein, wie Colzakuchen, Sonnenblume, Erdnuss, Baumwolle; und
- Proteine von Knollen, wie insbesondere Kartoffel, Maniok.

23. Verfahren zur Herstellung von biologisch abbaubaren oder erneuerbaren Proteinmaterialien nach Anspruch 22, **dadurch gekennzeichnet, dass** die Vernetzung darin besteht, dass:
1) die Proteine und mindestens eine Cetose von 3 bis 5 Kohlenstoffatomen mit einer Cetose-Konzentration in Trockengewicht von 0,5 bis 20 %, vorzugsweise 1 bis 10 % und noch bevorzugter 1 bis 5 %, bezogen auf das Trockengewicht der zu behandelnden Rohproteine, in Kontakt gebracht werden,
2) der Kontakt zwischen den Proteinen und der Cetose während einer Zeit von mehr als einigen Minuten, vorzugsweise zwischen 10 Minuten bis 60 Minuten, vorzugsweise während 30 Minute, bei einer Temperatur zwischen 20 und 105°C, vorzugsweise zwischen 35 und 105°C, aufrechterhalten wird,
3) und die auf diese Weise erhaltenen vernetzten Proteine gewonnen werden.

24. Verfahren nach einem der Ansprüche 22 bis 23, **dadurch gekennzeichnet, dass** die Cetose von 3 bis 5 Kohlenstoffatomen aus der Gruppe ausgewählt ist, die aus Dihydroxyaceton, Erythrulose, Ribulose und Xylulose besteht, und vorzugsweise Dihydroxyaceton ist.

25. Verfahren nach einem der Ansprüche 22 bis 24, **dadurch gekennzeichnet, dass** die vernetzten Proteine darin die mit mineralischen Gerbstoffen gerbstoffbehandelten Proteine, wie insbesondere die mit Chrom gerbstoffbehandelten Proteine, substituieren.

26. Verfahren nach einem der Ansprüche 22 bis 24, **dadurch gekennzeichnet, dass** die vernetzten Proteine darin die mit aldehydischen Gerbstoffen gerbstoffbehandelten Proteine, wie insbesondere die mit Formaldehyd, mit Glyoxal oder mit Glutaraldehyd gerbstoffbehandelten Proteine, und insbesondere die mit Formol gerbstoffbehandelten Proteine substituieren.

27. Verfahren nach einem der Ansprüche 22 bis 26, **dadurch gekennzeichnet, dass** das biologisch abbaubare oder erneuerbare Proteinmaterial ausgewählt ist aus Kunststofffilmen, Ledern, Textilfasern, Gelatinekapseln, Kapseln oder Nahrungsmittel-Proteinzusammensetzungen.

28. Verwendung, für die Herstellung von biologisch abbaubaren oder erneuerbaren Proteinmaterialien, von - Proteine von tierischen Geweben, Milch, Blut, wie insbesondere Casein, Gelatine, Collagen,
- Getreideproteine, wie insbesondere Mais-, Weizen-, Reisproteine;
- Eiweißpflanzenproteine, wie insbesondere Proteine von Erbsen, Luzerne, Lupine, Gerste, Hirse, Sorgum;
- Ölsaatenproteine, wie insbesondere Proteine von Soja, wie Sojakuchen, Colza, Lein, wie Colzakuchen, Sonnenblume, Erdnuss, Baumwolle; und
- Proteine von Knollen, wie insbesondere Kartoffel, Maniok.

29. Verwendung nach Anspruch 28, **dadurch gekennzeichnet, dass** das Vernetzungsverfahren darin besteht, dass:
1) die Proteine und mindestens eine Cetose von 3 bis 5 Kohlenstoffatomen mit einer Cetose-Konzentration in Trockengewicht von 0,5 bis 20 %, vorzugsweise 1 bis 10 % und noch bevorzugter 1 bis 5 %, bezogen auf das Trockengewicht der zu behandelnden Rohproteine, in Kontakt gebracht werden,
2) der Kontakt zwischen den Proteinen und der Cetose während einer Zeit von mehr als einigen Minuten, vorzugsweise zwischen 10 Minuten bis 60 Minuten, vorzugsweise während 30 Minute, bei einer Temperatur zwischen 20 und 105°C, vorzugsweise zwischen 35 und 105°C, aufrechterhalten wird,
3) und die auf diese Weise erhaltenen vernetzten Proteine gewonnen werden.

30. Verwendung nach einem der Ansprüche 28 bis 29, **dadurch gekennzeichnet, dass** die Cetose von 3 bis 5 Kohlenstoffatomen aus der Gruppe ausgewählt ist, die aus Dihydroxyaceton, Erythrulose, Ribulose und Xylulose besteht, und vorzugsweise Dihydroxyaceton ist.

31. Verwendung nach einem der Ansprüche 28 bis 30, **dadurch gekennzeichnet, dass** die vernetzten Proteine darin die mit mineralischen Gerbstoffen gerbstoffbehandelten Proteine, wie insbesondere die mit Chrom gerbstoffbehandelten Proteine, substituieren.

32. Verwendung nach einem der Ansprüche 28 bis 30, **dadurch gekennzeichnet, dass** die vernetzten Proteine darin die mit aldehydischen Gerbstoffen gerbstoffbehandelten Proteine, wie insbesondere die mit Formaldehyd, mit Glyoxal oder mit Glutaraldehyd gerbstoffbehandelten Proteine, und insbesondere die mit Formol gerbstoffbehandelten Proteine substituieren.

33. Verfahren nach einem der Ansprüche 28 bis 32, **dadurch gekennzeichnet, dass** das biologisch abbaubare oder erneuerbare Proteinmaterial ausgewählt ist aus Kunststofffilmen, Ledern, Textilfasern, Gelatinekapseln, Kapseln oder Nahrungsmittel-Proteinzusammensetzungen.
